(19) Europäisches Patentamt / European Patent Office / Office européen des brevets

(11) **EP 3 601 553 B1**

(12) **EUROPEAN PATENT SPECIFICATION**

(45) Date of publication and mention
of the grant of the patent:
**03.12.2025 Bulletin 2025/49**

(21) Application number: **18718664.8**

(22) Date of filing: **02.04.2018**

(51) International Patent Classification (IPC):
*C12N 9/28* (2006.01)    *C11D 3/386* (2006.01)

(52) Cooperative Patent Classification (CPC):
**C12N 9/2417; C11D 3/386;** Y02E 50/10

(86) International application number:
**PCT/US2018/025679**

(87) International publication number:
**WO 2018/184004 (04.10.2018 Gazette 2018/40)**

(54) **ALPHA-AMYLASE COMBINATORIAL VARIANTS**

KOMBINATORISCHE ALPHA-AMYLASE-VARIANTEN

VARIANTS COMBINATOIRES D'ALPHA-AMYLASES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **31.03.2017  US 201762479726 P**

(43) Date of publication of application:
**05.02.2020 Bulletin 2020/06**

(73) Proprietor: **Danisco US Inc.**
**Palo Alto, California 94304 (US)**

(72) Inventors:
• **LASSILA, Jonathan**
**Palo Alto**
**California 94304 (US)**
• **RAMER, Sandra W.**
**Palo Alto**
**California 94304 (US)**

(74) Representative: **Mewburn Ellis LLP**
**Aurora Building**
**Counterslip**
**Bristol BS1 6BX (GB)**

(56) References cited:
WO-A1-01/64852         WO-A1-2013/184577
WO-A1-2014/164777      WO-A2-2013/063460
US-A1- 2016 053 243

• **KAZUAKI IGARASHI ET AL: "IMPROVED
THERMOSTABILITY OF A BACILLUS ALPHA-
AMYLASE BY DELETION OFAN ARGININE-
GLYCINE RESIDUE IS CAUSED BY ENHANCED
CALCIUM BINDING", BIOCHEMICAL AND
BIOPHYSICAL RESEARCH COMMUNICATIONS,
ELSEVIER, AMSTERDAM, NL, vol. 248, no. 2, 1
January 1998 (1998-01-01), pages 372 - 377,
XP002901159, ISSN: 0006-291X, DOI: 10.1006/
BBRC.1998.8970**

## Description

### FIELD OF THE INVENTION

Disclosed are compositions and methods relating to variant α-amylases containing a plurality of combinable mutations. The variant α-amylases are useful, for example, for starch liquefaction and saccharification, cleaning starchy stains, textile desizing, baking, and brewing.

### BACKGROUND

Starch consists of a mixture of amylose (15-30% w/w) and amylopectin (70-85% w/w). Amylose consists of linear chains of α-1,4-linked glucose units having a molecular weight (MW) from about 60,000 to about 800,000. Amylopectin is a branched polymer containing α-1,6 branch points every 24-30 glucose units; its MW may be as high as 100 million.

Sugars from starch, in the form of concentrated dextrose syrups, are currently produced by an enzyme catalyzed process involving: (1) gelatinization and liquefaction (or viscosity reduction) of solid starch with an α-amylase into dextrins having an average degree of polymerization of about 7-10, and (2) saccharification of the resulting liquefied starch (*i.e.* starch hydrolysate) with amyloglucosidase (also called glucoamylase or GA). The resulting syrup has a high glucose content. Much of the glucose syrup that is commercially produced is subsequently enzymatically isomerized to a dextrose/fructose mixture known as isosyrup. The resulting syrup also may be fermented with microorganisms, such as yeast, to produce commercial products including ethanol, citric acid, lactic acid, succinic acid, itaconic acid, monosodium glutamate, gluconates, lysine, other organic acids, other amino acids, and other biochemicals, for example. Fermentation and saccharification can be conducted simultaneously (*i.e.*, an SSF process) to achieve greater economy and efficiency.

α-amylases hydrolyze starch, glycogen, and related polysaccharides by cleaving internal α-1,4-glucosidic bonds at random. α-amylases, particularly from Bacilli, have been used for a variety of different purposes, including starch liquefaction and saccharification, textile desizing, starch modification in the paper and pulp industry, brewing, baking, production of syrups for the food industry, production of feedstocks for fermentation processes, and in animal feed to increase digestability. These enzymes can also be used to remove starchy soils and stains during dishwashing and laundry washing.

Numerous publications have described mutations in α-amylases. However, not all mutations produce the same effect in different molecules and not all mutations can be combined. In addition, many mutations produce molecules that have certain desirable qualities at the expense of other properties. The need exists for robust engineered α-amylases molecules.

WO 2001/64852 A1 relates to polypeptides having α-amylase activity as well as nucleotide sequences encoding these polypeptides. Igarahi et al (1998) Biochemical and Biophysical Research Communciations. 248: 372-377 reports that improved thermostability of Bacillus α-amylase by deletion of Arg181-Gly182 is due to enhanced calcium binding to the enzyme. WO 2013/063460 A2 relates to variant maltohexaose forming α-amylases and their various uses. WO 2013/184577 A1 relates to variants derived from the α-amylase of cytophaga sp. Amylases and their various uses. WO 2014/164777 A1 and US 2016/053243 A1 relate to variant α-amylases containing combinable mutations, as well as their various uses.

### SUMMARY

The present compositions and methods relate to variant amylase polypeptides, and methods of use, thereof. Aspects and embodiments of the present compositions and methods are summarized in the following separately-numbered paragraphs:

1. 1. A recombinant variant of a parent α-amylase comprising:

(a) a deletion of amino acid residues corresponding to R181 and G182, using SEQ ID NO: 1; and the mutations Q172R, A186G and 1324M; wherein the variant α-amylase or the parent α-amylase has at least 91%, optionally 92%, optionally 93%, optionally 94%, optionally 95%, optionally 96%, optionally 97%, optionally 98%, or optionally 99%, amino acid sequence identity relative to SEQ ID NO: 1, which is used for numbering; and wherein the variant has improved cleaning performance in automatic dishwashing compared to the parent α-amylase,

wherein said parent α-amylase differs from the variant α-amylase only by the absence of said deletion and mutations.
2. In some embodiments, the variant α-amylase of paragraph 1 has at least 95% amino acid sequence identity relative to SEQ ID NO: 1.

3. In some embodiments, the variant α-amylase of paragraph 1, comprises the amino acid sequence of SEQ ID NO:1 with deletion of amino acid residues corresponding to R181 and G182 and the mutations Q172R, A186G and I324M.

4. In another aspect, a polynucleotide encoding the variant amylase of any of paragraphs 1-3, an expression vector comprising the polynucleotide, or an expression host comprising the polynucleotide or the expression vector is provided.

5. In another aspect, a composition for liquefying starch comprising the variant amylase of any of paragraphs 1-3 is provided.

6. In another aspect, a detergent composition comprising the variant amylase of any of paragraphs 1-3 is provided.

7. In another aspect, a method for converting starch to oligosaccharides, comprising contacting starch with effective amount of the variant amylase of any of the paragraphs 1-3 is provided.

8. In another aspect, a method is provided for removing a starchy stain or soil from a surface, comprising contacting the surface with an effective amount of the variant amylase of any of the paragraphs 1-3, and allowing the polypeptide to hydrolyze starch components present in the starchy stain to produce smaller starch-derived molecules that dissolve in the aqueous composition, thereby removing the starchy stain from the surface.

These and other aspects and embodiments of the compositions and methods will be apparent from the present description and drawings.

## BRIEF DESCRIPTION OF THE DRAWINGS

Figure 1 is a graph showing the starch cleaning performance of enzymes in CASCADE® PLATINUM™ dishwashing formula with inactivated enzyme.

Figure 2 is a graph showing the starch cleaning performance of enzymes in CASCADE® PLATINUM™ dishwashing formula with inactivated enzyme.

Figure 3 is a graph showing the starch cleaning performance of enzymes in CASCADE® PLATINUM™ dishwashing formula with inactivated enzyme.

Figure 4 is a graph showing the starch cleaning performance of enzymes in FINISH® QUANTUM™ dishwashing formula with inactivated enzyme.

## DETAILED DESCRIPTION

Described are compositions and methods relating to variant α-amylase enzymes. Exemplary applications for the variant amylase enzymes are for starch liquefaction and saccharification, for cleaning starchy stains in laundry, dishwashing, and other applications, for textile processing (*e.g.*, desizing), in animal feed for improving digestibility, and and for baking and brewing. These and other aspects of the compositions and methods are described in detail, below.

Prior to describing the various aspects and embodiments of the present compositions and methods, the following definitions and abbreviations are described.

### 1. Definitions and Abbreviations

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art. The following terms are provided below.

### 1.1. Abbreviations and Acronyms

The following abbreviations/acronyms have the following meanings unless otherwise specified:

DNA          deoxyribonucleic acid

| EC | Enzyme Commission |
|----|----|
| FH | French hardness |
| GA | glucoamylase |
| GH | general hardness |
| HDL | high density liquid detergent |
| HDD | heavy duty powder detergent |
| HSG | high suds granular detergent |
| HFCS | high fructose corn syrup |
| IRS | insoluble residual starch |
| kDa | kiloDalton |
| MW | molecular weight |
| MWU | modified Wohlgemuth unit; $1.6 \times 10^{-5}$ mg/MWU = unit of activity |
| NCBI | National Center for Biotechnology Information |
| PI | performance index |
| ppm | parts per million, *e.g.,* $\mu$g protein per gram dry solid |
| RCF | relative centrifugal/centripetal force (*i.e.*, x gravity) |
| RNA | ribonucleic acid |
| sp. | species |
| Tm | melting temperature |
| w/v | weight/volume |
| w/w | weight/weight |
| v/v | volume/volume |
| wt% | weight percent |
| °C | degrees Centigrade |
| $H_2O$ | water |
| $dH_2O$ or DI | deionized water |
| $dIH_2O$ | deionized water, Milli-Q filtration |
| g or gm | grams |
| $\mu$g | micrograms |
| mg | milligrams |
| kg | kilograms |
| $\mu$L and $\mu$l | microliters |
| mL and ml | milliliters |
| mm | millimeters |
| $\mu$m | micrometer |
| M | molar |
| mM | millimolar |
| $\mu$M | micromolar |
| U | units |
| sec | seconds |
| min(s) | minute/minutes |
| hr(s) | hour/hours |
| ETOH | ethanol |
| N | normal |
| MWCO | molecular weight cut-off |
| CAZy | Carbohydrate-Active Enzymes database |

## 1.2. Definitions

The terms "amylase" or "amylolytic enzyme" refer to an enzyme that is, among other things, capable of catalyzing the degradation of starch. $\alpha$-amylases are hydrolases that cleave the $\alpha$-D-(1$\rightarrow$4) O-glycosidic linkages in starch. Generally, $\alpha$-amylases (EC 3.2.1.1; $\alpha$-D-(1$\rightarrow$4)-glucan glucanohydrolase) are defined as endo-acting enzymes cleaving $\alpha$-D-(1$\rightarrow$4) O-glycosidic linkages within the starch molecule in a random fashion yielding polysaccharides containing three or more (1-4)-$\alpha$-linked D-glucose units. In contrast, the exo-acting amylolytic enzymes, such as $\beta$-amylases (EC 3.2.1.2; $\alpha$-D-(1$\rightarrow$4)-glucan maltohydrolase) and some product-specific amylases like maltogenic $\alpha$-amylase (EC 3.2.1.133) cleave the polysaccharide molecule from the non-reducing end of the substrate. $\beta$-amylases, $\alpha$-glucosidases (EC 3.2.1.20; $\alpha$-D-glucoside glucohydrolase), glucoamylase (EC 3.2.1.3; $\alpha$-D-(1$\rightarrow$4)-glucan glucohydrolase), and product-specific amylases like the maltotetraosidases (EC 3.2.1.60) and the maltohexaosidases (EC 3.2.1.98) can produce malto-oligosac-

4

charides of a specific length or enriched syrups of specific maltooligosaccharides.

The term "starch" refers to any material comprised of the complex polysaccharide carbohydrates of plants, comprised of amylose and amylopectin with the formula $(C_6H_{10}O_5)_x$, wherein X can be any number. The term includes plant-based materials such as grains, cereal, grasses, tubers and roots, and more specifically materials obtained from wheat, barley, corn, rye, rice, sorghum, brans, cassava, millet, milo, potato, sweet potato, and tapioca. The term "starch" includes granular starch. The term "granular starch" refers to raw, *i.e.*, uncooked starch, *e.g.*, starch that has not been subject to gelatinization.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

Reference to the wild-type polypeptide is understood to include the mature form of the polypeptide. A "mature" polypeptide or variant, thereof, is one in which a signal sequence is absent, for example, cleaved from an immature form of the polypeptide during or following expression of the polypeptide.

The term "variant," with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

In the case of the present α-amylases, "activity" refers to α-amylase activity, which can be measured as described, herein.

The term "performance benefit" refers to an improvement in a desirable property of a molecule. Exemplary performance benefits include, but are not limited to, increased hydrolysis of a starch substrate, increased grain, cereal or other starch substrate liquifaction performance, increased cleaning performance, increased thermal stability, increased detergent stability, increased storage stability, increased solubility, an altered pH profile, decreased calcium dependence, increased specific activity, modified substrate specificity, modified substrate binding, modified pH-dependent activity, modified pH-dependent stability, increased oxidative stability, and increased expression. In some cases, the performance benefit is realized at a relatively low temperature. In some cases, the performance benefit is realized at relatively high temperature.

The terms "protease" and "proteinase" refer to an enzyme protein that has the ability to perform "proteolysis" or "proteolytic cleavage" which refers to hydrolysis of peptide bonds that link amino acids together in a peptide or polypeptide chain forming the protein. This activity of a protease as a protein-digesting enzyme is referred to as "proteolytic activity."

The terms "serine protease" refers to enzymes that cleave peptide bonds in proteins, in which enzymes serine serves as the nucleophilic amino acid at the enzyme active site. Serine proteases fall into two broad categories based on their structure: chymotrypsin-like (trypsin-like) or subtilisin-like. Most commonly used in laundry and dishwashing detergents are serine protease, particularly subtlisins.

"Combinatorial variants" are variants comprising two or more mutations, *e.g.*, 2, 3, 4, 5, 6, 7, 8, 9, 10, or more, substitutions, deletions, and/or insertions.

"Combinable mutations" are mutations at any amino acid position that can be used to make combinatorial variants. Combinable mutations improve at least one desired property of the molecule (in this case, an amylase), while not significantly decreasing either expression, activity, or stability.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e.g.*, a heterologous promoter in an expression vector. Recombinant proteins may differ from a native sequence by one or more amino acids and/or are fused with heterologous sequences. A vector comprising a nucleic acid encoding an amylase is a recombinant vector.

The terms "recovered," "isolated," and "separated," refer to a compound, protein (polypeptides), cell, nucleic acid, amino acid, or other specified material or component that is removed from at least one other material or component with which it is naturally associated as found in nature. An "isolated" polypeptides, thereof, includes, but is not limited to, a culture broth containing secreted polypeptide expressed in a heterologous host cell.

The term "purified" refers to material (*e.g.*, an isolated polypeptide or polynucleotide) that is in a relatively pure state, *e.g.,* at least about 90% pure, at least about 95% pure, at least about 98% pure, or even at least about 99% pure.

The term "enriched" refers to material (*e.g.*, an isolated polypeptide or polynucleotide) that is in about 50% pure, at least about 60% pure, at least about 70% pure, or even at least about 70% pure.

The terms "thermostable" and "thermostability," with reference to an enzyme, refer to the ability of the enzyme to retain activity after exposure to an elevated temperature. The thermostability of an enzyme, such as an amylase enzyme, is measured by its half-life (t1/2) given in minutes, hours, or days, during which half the enzyme activity is lost under defined conditions. The half-life may be calculated by measuring residual α-amylase activity following exposure to (*i.e.*, challenge by) an elevated temperature.

A "pH range," with reference to an enzyme, refers to the range of pH values under which the enzyme exhibits catalytic activity.

The terms "pH stable" and "pH stability," with reference to an enzyme, relate to the ability of the enzyme to retain activity over a wide range of pH values for a predetermined period of time (*e.g.*, 15 min., 30 min., 1 hour).

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.*, N→C).

The term "nucleic acid" encompasses DNA, RNA, heteroduplexes, and synthetic molecules capable of encoding a polypeptide. Nucleic acids may be single stranded or double stranded, and may contain chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

"Hybridization" refers to the process by which one strand of nucleic acid forms a duplex with, *i.e.*, base pairs with, a complementary strand, as occurs during blot hybridization techniques and PCR techniques. Stringent hybridization conditions are exemplified by hybridization under the following conditions: 65°C and 0.1X SSC (where 1X SSC = 0.15 M NaCl, 0.015 M Na3 citrate, pH 7.0). Hybridized, duplex nucleic acids are characterized by a melting temperature (Tm), where one half of the hybridized nucleic acids are unpaired with the complementary strand. Mismatched nucleotides within the duplex lower the Tm. A nucleic acid encoding a variant α-amylase may have a Tm reduced by 1°C - 3°C or more compared to a duplex formed between the nucleotide of SEQ ID NO: 2 and its identical complement.

A "synthetic" molecule is produced by in vitro chemical or enzymatic synthesis rather than by an organism.

The terms "transformed," "stably transformed," and "transgenic," used with reference to a cell means that the cell contains a non-native (*e.g.*, heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g.*, an amylase) has been introduced. Exemplary host strains are microorganism cells (*e.g.*, bacteria, filamentous fungi, and yeast) capable of expressing the polypeptide of interest and/or fermenting saccharides. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g.*, hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

"Biologically active" refer to a sequence having a specified biological activity, such an enzymatic activity.

The term "specific activity" refers to the number of moles of substrate that can be converted to product by an enzyme or enzyme preparation per unit time under specific conditions. Specific activity is generally expressed as units (U)/mg of protein.

As used herein, "water hardness" is a measure of the minerals (e.g., calcium and magnesium) present in water.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The smaller swatch can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of metal, plastic, glass, ceramic, or another suitable material that is coated with the soil substrate. The one or more coated beads are then placed into wells of 96-, 48-, or 24-well plates or larger formats, containing suitable buffer and enzyme.

"A cultured cell material comprising an amylase" or similar language, refers to a cell lysate or supernatant (including media) that includes an amylase as a component. The cell material may be from a heterologous host that is grown in culture for the purpose of producing the amylase.

"Percent sequence identity" means that a particular sequence has at least a certain percentage of amino acid residues identical to those in a specified reference sequence, when aligned using the CLUSTAL W algorithm with default parameters. See Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence.

"Fused" polypeptide sequences are connected, *i.e.*, operably linked, via a peptide bond between two subject polypeptide sequences.

The term "filamentous fungi" refers to all filamentous forms of the subdivision Eumycotina, particulary Pezizomycotina species.

The term "dry solids content" (ds) refers to the total solids of a slurry in a dry weight percent basis. The term "slurry" refers to an aqueous mixture containing insoluble solids.

The phrase "simultaneous saccharification and fermentation (SSF)" refers to a process in the production of biochemicals in which a microbial organism, such as an ethanologenic microorganism, and at least one enzyme, such as an amylase, are present during the same process step. SSF includes the contemporaneous hydrolysis of starch substrates (granular, liquefied, or solubilized) to saccharides, including glucose, and the fermentation of the saccharides into alcohol or other biochemical or biomaterial in the same reactor vessel.

An "ethanologenic microorganism" refers to a microorganism with the ability to convert a sugar or oligosaccharide to ethanol.

The term "fermented beverage" refers to any beverage produced by a method comprising a fermentation process, such as a microbial fermentation, *e.g.*, a bacterial and/or fungal fermentation. "Beer" is an example of such a fermented beverage, and the term "beer" is meant to comprise any fermented wort produced by fermentation/brewing of a starch-containing plant material. Often, beer is produced exclusively from malt or adjunct, or any combination of malt and adjunct.

The term "malt" refers to any malted cereal grain, such as malted barley or wheat.

The term "adjunct" refers to any starch and/or sugar containing plant material that is not malt, such as barley or wheat malt. Examples of adjuncts include common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, cassava and syrups, such as corn syrup, sugar cane syrup, inverted sugar syrup, barley and/or wheat syrups, and the like.

The term "mash" refers to an aqueous slurry of any starch and/or sugar containing plant material, such as grist, *e.g.*, comprising crushed barley malt, crushed barley, and/or other adjunct or a combination thereof, mixed with water later to be separated into wort and spent grains.

The term "wort" refers to the unfermented liquor run-off following extracting the grist during mashing.

The term "about" refers to ± 15% to the referenced value.

## 2. α-Amylase variants

An aspect of the present compositions and methods are variant α-amylase enzymes that include combinations of mutations that improve their performance in industrial applications, as defined in the claims. The combinatorial variants are based on an α-amylase from a *Bacillus* sp., herein, refered to as "BspAmy24." The amino acid sequence of the mature form of the BspAmy24 α-amylase polypeptide is shown below as SEQ ID NO: 1:

```
HHNGTNGTMMQYFEWHLPNDGQHWNRLRNDAANLKNLGITAVWIPPAWKGTSQNDVGYGAYDLY

DLGEFNQKGTIRTKYGTRSQLQSAIASLQNNGIQVYGDVVMNHKGGADGTEWVQAVEVNPSNRN

QEVTGEYTIEAWTKFDFPGRGNTHSSFKWRWYHFDGTDWDQSRQLNNRIYKFRGTGKAWDWEVD

TENGNYDYLMYADVDMDHPEVINELRRWGVWYTNTLNLDGFRIDAVKHIKYSFTRDWLNHVRST

TGKNNMFAVAEFWKNDLGAIENYLHKTNWNHSVFDVPLHYNLYNASKSGGNYDMRQILNGTVVS

KHPIHAVTFVDNHDSQPAEALESFVEAWFKPLAYALILTREQGYPSVFYGDYYGIPTHGVAAMK

GKIDPILEARQKYAYGTQHDYLDHHNIIGWTREGNSAHPNSGLATIMSDGPGGSKWMYVGRHKA

GQVWRDITGNRTGTVTINADGWGNFSVNGGSVSIWVNK
```

Using SEQ ID NO: 1 as a starting point, a number of combinatorial variants were made and tested with a particular emphasis on identifying variants with a high level of cleaning performace under North American automatic dishwashing (ADW) conditions. Several variants that had excellent cleaning performance were identified.

Some, but not all, of the best performing variant BspAmy24 α-amylases included a deletion in the $X_1G/S_1X_2G_2$ motif adjacent to the calcium-binding loop corresponding to R181, G182, T183, and G184, using SEQ ID NO: 1 for numbering (underlined, above). Pair-wise deletions of amino acid residues corresponding to R181 and G182 or T183 and G184 are generally equally effective in improving stability and reducing calcium dependence because of the nature of the $X_1G/S_1X_2G_2$ motif. A deletion of amino acid residues corresponding to R181 and G182 may be referred to as "ΔRG," while a deletion of amino acid residues corresponding to T183 and G184 may be referred to as "ΔTG."

Other mutations present in the best performing variants are at the positions shown, below, using SEQ ID NO: 1 for numbering:

T183, E190, M202, Q172, A186, and/or I324, for example,

$$T183 + E190,$$

$$T183 + M202,$$

$$T183 + E190 + M202,$$

$$Q172 + A186 + I324$$

and

$$A186 + I324.$$

Specific mutations present in the best performing variants are shown, below, using SEQ ID NO: 1 for numbering: T183D, E190P, M202L, Q172R, A186G, and/or I324M, for example,

$$T183D + E190P,$$

$$T183D + M202L,$$

$$T183D + E190P + M202L,$$

$$Q172R + A186G + I324M$$

and

$$A186G + I324M.$$

These combinations of mutations can be used in conjunction with the aforementioned deletions at positions corresponding to R181, G182, T183, and/or G184. The substitution T183D may have an impact on solubilty but is not believed to significantly contribute to performance. The aforementioned mutations can likely be combined with other mutations described in $\alpha$-amylases, for which there is a substantial amount of published patent literature.

In some embodiments, the present $\alpha$-amylase variants have the indicated combinations of mutations as defined in the claims, and a defined degree of amino acid sequence homology/identity to SEQ ID NO: 1, the defined degree being at least 91%, at least 92%, at least 93%, at least 94%, at least 95%, at least 96%, at least 97%, at least 98% or even at least 99% amino acid sequence homology/identity.

In some embodiments, the present $\alpha$-amylase variants have the indicated combinations of mutations as defined in the claims and are derived from a parental amylase having a defined degree of amino acid sequence homology/identity to SEQ ID NO: 1, the defined degree being, at least 91%, at least 92%, at least 93%, at least 94%, at least 95%, at least 96%, at least 97%, at least 98% or even at least 99% amino acid sequence homology/identity.

Furthermore, the present amylases may include any number of conservative amino acid substitutions, provided they are still encompassed by the claims. Exemplary conservative amino acid substitutions are listed in Table 1

**Table 1.** Conservative amino acid substitutions

| For Amino Acid | Code | Replace with any of |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |

(continued)

| For Amino Acid | Code | Replace with any of |
|---|---|---|
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

The reader will appreciate that some of the above mentioned conservative mutations can be produced by genetic manpulation, while others are produced by introducing synthetic amino acids into a polypeptide by genetic or other means.

The present amylase may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective amylase polypeptides. The present amylase polypeptides may also be truncated to remove the N or C-termini, so long as the resulting polypeptides retain amylase activity.

The present amylase may be a "chimeric" or "hybrid" polypeptide, in that it includes at least a portion of a first amylase polypeptide, and at least a portion of a second amylase polypeptide (such chimeric amylases have recently been "rediscovered" as domain-swap amylases). The present amylases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

## 2.5. Nucleotides encoding variant amylase polypeptides

In another aspect, nucleic acids encoding a variant amylase polypeptide defined by the claims are provided.

It will be appreciated that due to the degeneracy of the genetic code, a plurality of nucleic acids may encode the same polypeptide.

The nucleic acid may hybridize under stringent or very stringent conditions to a nucleic acid encoding (or complementary to a nucleic acid encoding) an amylase defined by the claims.

The nucleic acid may hybridize under stringent or very stringent conditions to the nucleic acid of SEQ ID NO: 2 or the complement, thereof:

```
CACCATAATGGTACGAATGGAACGATGATGCAATACTTTGAGTGGCATTTACCAAATGATGGTC

AGCATTGGAATCGGTTAAGAAATGATGCCGCAAATTTAAAGAATCTAGGAATTACAGCAGTTTG

GATTCCACCTGCTTGGAAAGGCACATCACAAAATGATGTCGGCTACGGGGCTTATGATTTATAT

GACCTTGGTGAATTTAATCAAAAAGGAACAATTCGAACGAAATATGGTACGCGGAGTCAATTAC

AATCAGCAATTGCCTCATTACAAAATAATGGTATTCAAGTTTACGGCGATGTTGTTATGAATCA
```

```
CAAAGGCGGTGCAGATGGAACTGAATGGGTTCAAGCTGTTGAAGTAAATCCAAGTAACCGCAAT
CAAGAAGTGACTGGCGAATATACAATTGAAGCATGGACAAAATTCGATTTTCCAGGGAGAGGGA
ATACGCATTCAAGTTTTAAATGGAGATGGTACCATTTTGATGGAACAGATTGGGACCAATCTCG
GCAACTTAACAATCGCATCTATAAATTCCGCGGGACCGGAAAGCATGGGACTGGGAAGTTGAT
ACGGAGAACGGAAATTATGATTACTTAATGTATGCTGATGTTGATATGGATCATCCAGAAGTAA
TTAATGAACTAAGGAGATGGGGTGTTTGGTACACAAATACACTAAATCTTGATGGATTCAGAAT
TGACGCTGTTAAACATATTAAATATAGTTTTACTAGAGACTGGCTAAATCATGTAAGAAGTACA
ACTGGTAAGAATAATATGTTCGCTGTGGCAGAGTTTTGGAAAAATGACTTAGGTGCCATTGAAA
ATTACTTACACAAAACGAACTGGAATCACTCTGTATTCGATGTTCCACTACATTACAACCTTTA
TAATGCGTCTAAAGTGGTGGTAATTACGATATGCGTCAGATTTTAAATGGTACAGTAGTATCA
AAACATCCAATACATGCTGTTACTTTTGTTGATAATCATGATTCTCAACCTGCAGAAGCATTGG
AATCGTTTGTTGAAGCGTGGTTTAAACCATTAGCTTATGCGCTTATCTTAACACGCGAGCAAGG
ATATCCTTCGGTGTTTTATGGAGATTATTATGGAATTCCTACTCATGGAGTAGCTGCAATGAAA
GGTAAGATTGACCCTATTTTAGAAGCACGTCAAAAATATGCATATGGAACACAGCATGATTATT
TAGATCATCATAATATCATTGGTTGGACACGAGAAGGAAATTCAGCACACCCTAATTCCGGTCT
TGCAACGATTATGTCCGATGGCCCTGGTGGTAGTAAATGGATGTATGTTGGTCGTCATAAAGCA
GGCCAAGTGTGGCGTGATATTACTGGTAACAGAACAGGCACTGTTACGATAAATGCCGATGGTT
GGGGTAATTTCTCCGTTAATGGTGGATCTGTATCGATTTGGGTAAATAAA
```

### 3. Production of Variant Amylases

The present variant amylases can be produced in host cells, for example, by secretion or intracellular expression, using methods well-known in the art. Fermentation, separation, and concentration techniques are well known in the art and conventional methods can be used to prepare a concentrated, variant-$\alpha$-amylase-polypeptide-containing solution.

For production scale recovery, variant $\alpha$-amylase polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

### 4. Compositions and Uses of Variant Amylases

Variant amylases are useful for a variety of industrial applications. For example, variant amylases are useful in a starch conversion process, particularly in a saccharification process of a starch that has undergone liquefaction. The desired end-product may be any product that may be produced by the enzymatic conversion of the starch substrate. For example, the desired product may be a syrup rich in glucose and maltose, which can be used in other processes, such as the preparation of HFCS, or which can be converted into a number of other useful products, such as ascorbic acid intermediates (*e.g.*, gluconate; 2-keto-L-gulonic acid; 5-keto-gluconate; and 2,5-diketogluconate); 1,3-propanediol; aromatic amino acids (*e.g.*, tyrosine, phenylalanine and tryptophan); organic acids (*e.g.*, lactate, pyruvate, succinate, isocitrate, and oxaloacetate); amino acids (*e.g.*, serine and glycine); antibiotics; antimicrobials; enzymes; vitamins; and hormones.

The starch conversion process may be a precursor to, or simultaneous with, a fermentation process designed to produce alcohol for fuel or drinking (*i.e.*, potable alcohol). One skilled in the art is aware of various fermentation conditions that may be used in the production of these end-products. Variant amylases are also useful in compositions and methods of food preparation. These various uses of variant amylases are described in more detail below.

### 4.1. Preparation of Starch Substrates

Those of general skill in the art are well aware of available methods that may be used to prepare starch substrates for use in the processes disclosed herein. For example, a useful starch substrate may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains 70-72% starch. Specifically contemplated starch substrates are corn starch and wheat starch. The starch from a grain may be ground or whole and includes corn solids, such as kernels, bran and/or cobs. The starch may also be highly refined raw starch or feedstock from starch refinery processes. Various starches also are commercially available. For example, corn starch is available from Cerestar, Sigma, and Katayama Chemical Industry Co. (Japan); wheat starch is available from Sigma; sweet potato starch is available from Wako Pure Chemical Industry Co. (Japan); and potato starch is available from Nakaari Chemical Pharmaceutical Co. (Japan).

The starch substrate can be a crude starch from milled whole grain, which contains non-starch fractions, *e.g.*, germ residues and fibers. Milling may comprise either wet milling or dry milling or grinding. In wet milling, whole grain is soaked in water or dilute acid to separate the grain into its component parts, *e.g.*, starch, protein, germ, oil, kernel fibers. Wet milling efficiently separates the germ and meal (*i.e.*, starch granules and protein) and is especially suitable for production of syrups. About 90% of the corn oil is in the germ. In dry milling or grinding, whole kernels are ground into a fine powder and often processed without fractionating the grain into its component parts. In some cases, oils and/or fiber from the kernels are recovered. Dry ground grain thus will comprise significant amounts of non-starch carbohydrate compounds, in addition to starch. Dry grinding of the starch substrate can be used for production of ethanol and other biochemicals. The starch to be processed may be a highly refined starch quality, for example, at least 90%, at least 95%, at least 97%, or at least 99.5% pure.

### 4.2. Gelatinization and Liquefaction of Starch

As used herein, the term "liquefaction" or "liquefy" means a process by which starch is converted to less viscous and shorter chain dextrins. Generally, this process involves gelatinization of starch simultaneously with or followed by the addition of an $\alpha$-amylase, although additional liquefaction-inducing enzymes optionally may be added. The starch substrate prepared as described above may be slurried with water. The starch slurry may contain starch as a weight percent of dry solids of about 10-55%, about 20-45%, about 30-45%, about 30-40%, or about 30-35%. $\alpha$-amylase may be added to the slurry, with a metering pump, for example. The $\alpha$-amylase typically used for this application is a thermally stable, bacterial $\alpha$-amylase, such as a *Geobacillus stearothermophilus* $\alpha$-amylase. The $\alpha$-amylase is usually supplied, for example, at about 1500 units per kg dry matter of starch. To optimize $\alpha$-amylase stability and activity, the pH of the slurry typically is adjusted to about pH 4.5-6.5 and about 1 mM of calcium (about 40 ppm free calcium ions) can also be added, depending upon the properties of the amylase used. Bacterial $\alpha$-amylase remaining in the slurry following liquefaction may be deactivated via a number of methods, including lowering the pH in a subsequent reaction step or by removing calcium from the slurry in cases where the enzyme is dependent upon calcium.

The slurry of starch plus the $\alpha$-amylase may be pumped continuously through a jet cooker, which is steam heated to 105°C. Gelatinization occurs rapidly under these conditions, and the enzymatic activity, combined with the significant shear forces, begins the hydrolysis of the starch substrate. The residence time in the jet cooker is brief. The partly gelatinized starch may be passed into a series of holding tubes maintained at 105-110°C and held for 5-8 min. to complete the gelatinization process ("primary liquefaction"). Hydrolysis to the required DE is completed in holding tanks at 85-95°C or higher temperatures for about 1 to 2 hours ("secondary liquefaction"). These tanks may contain baffles to discourage back mixing. As used herein, the term "minutes of secondary liquefaction" refers to the time that has elapsed from the start of secondary liquefaction to the time that the Dextrose Equivalent (DE) is measured. The slurry is then allowed to cool to room temperature. This cooling step can be 30 minutes to 180 minutes, *e.g.*, 90 minutes to 120 minutes. The liquefied starch typically is in the form of a slurry having a dry solids content (w/w) of about 10-50%; about 10-45%; about 15-40%; about 20-40%; about 25-40%; or about 25-35%.

Liquefaction with variant amylases advantageously can be conducted at low pH, eliminating the requirement to adjust the pH to about pH 5.5-6.5. Variants amylases can be used for liquefaction at a pH range of 2 to 7, *e.g.*, pH 3.0 - 7.5, pH 4.0 - 6.0, or pH 4.5 - 5.8. Variant amylases can maintain liquefying activity at a temperature range of about 85°C - 95°C, *e.g.*, 85°C, 90°C, or 95°C. For example, liquefaction can be conducted with 800 $\mu$g an amylase in a solution of 25% DS corn starch for 10 min at pH 5.8 and 85°C, or pH 4.5 and 95°C, for example. Liquefying activity can be assayed using any of a number of known viscosity assays in the art.

Starch liquifaction may be performed at a temperature range of 90-115°C, for the purpose of producing high-purity glucose syrups, HFCS, maltodextrins, etc.

### 4.3. Saccharification

The liquefied starch can be saccharified into a syrup that is rich in lower DP (*e.g.*, DP1 + DP2) saccharides, using variant amylases, optionally in the presence of another enzyme(s). The exact composition of the products of saccharification depends on the combination of enzymes used, as well as the type of granular starch processed. Advantageously, the syrup obtainable using the provided variant amylases may contain a weight percent of DP2 of the total oligosaccharides in the saccharified starch exceeding 30%, *e.g.*, 45% - 65% or 55% - 65%. The weight percent of (DP1 + DP2) in the saccharified starch may exceed about 70%, *e.g.*, 75% - 85% or 80% - 85%. The present amylases also produce a relatively high yield of glucose, *e.g.*, DP1 > 20%, in the syrup product.

Whereas liquefaction is generally run as a continuous process, saccharification is often conducted as a batch process. Saccharification typically is most effective at temperatures of about 60-65°C and a pH of about 4.0-4.5, *e.g.*, pH 4.3, necessitating cooling and adjusting the pH of the liquefied starch. The temperature and pH range can vary depending upon the properties of the enzymes. Saccharification may be performed, for example, at a temperature between about 40°C, about 50°C, or about 55°C to about 60°C or about 65°C. Saccharification is normally conducted in stirred tanks, which may take several hours to fill or empty. Enzymes typically are added either at a fixed ratio to dried solids as the tanks are filled or added as a single dose at the commencement of the filling stage. A saccharification reaction to make a syrup typically is run over about 24-72 hours, for example, 24-48 hours. When a maximum or desired DE has been attained, the reaction is stopped by heating to 85°C for 5 min., for example. Further incubation will result in a lower DE, eventually to about 90 DE, as accumulated glucose re-polymerizes to isomaltose and/or other reversion products via an enzymatic reversion reaction and/or with the approach of thermodynamic equilibrium. When using an amylase, saccharification optimally is conducted at a temperature range of about 30°C to about 75°C, *e.g.*, 45°C - 75°C or 47°C - 74°C. The saccharifying may be conducted over a pH range of about pH 3 to about pH 7, *e.g.*, pH 3.0 - pH 7.5, pH 3.5 - pH 5.5, pH 3.5, pH 3.8, or pH 4.5.

An $\alpha$-amylase may be added to the slurry in the form of a composition. An $\alpha$-amylase can be added to a slurry of a granular starch substrate in an amount of about 0.6 - 10 ppm ds, *e.g.*, 2 ppm ds. An $\alpha$-amylase can be added as a whole broth, clarified, enriched, partially purified, or purified enzyme. The specific activity of the amylase may be about 300 U/mg of enzyme, for example, measured with the PAHBAH assay. The $\alpha$-amylase also can be added as a whole broth product.

An $\alpha$-amylase may be added to the slurry as an isolated enzyme solution. For example, an $\alpha$-amylase can be added in the form of a cultured cell material produced by host cells expressing an amylase. An $\alpha$-amylase may also be secreted by a host cell into the reaction medium during the fermentation or SSF process, such that the enzyme is provided continuously into the reaction. The host cell producing and secreting amylase may also express an additional enzyme, such as a glucoamylase. For example, U.S. Patent No. 5,422,267 discloses the use of a glucoamylase in yeast for production of alcoholic beverages. For example, a host cell, *e.g.*, *Trichoderma reesei* or *Aspergillus niger*, may be engineered to co-express an $\alpha$-amylase and a glucoamylase, *e.g.*, HgGA, TrGA, or a TrGA variant, during saccharification. The host cell can be genetically modified so as not to express its endogenous glucoamylase and/or other enzymes, proteins or other materials. The host cell can be engineered to express a broad spectrum of various saccharolytic enzymes. For example, the recombinant yeast host cell can comprise nucleic acids encoding a glucoamylase, an alpha-glucosidase, an enzyme that utilizes pentose sugar, an $\alpha$-amylase, a pullulanase, an isoamylase, and/or an isopullulanase. *See, e.g.*, WO 2011/153516 A2.

### 4.4. Isomerization

The soluble starch hydrolysate produced by treatment with amylase can be converted into high fructose starch-based syrup (HFSS), such as high fructose corn syrup (HFCS). This conversion can be achieved using a glucose isomerase, particularly a glucose isomerase immobilized on a solid support. The pH is increased to about 6.0 to about 8.0, *e.g.*, pH 7.5 (depending on the isomerase), and $Ca^{2+}$ is removed by ion exchange. Suitable isomerases include SWEETZYME®, IT (Novozymes A/S); G-ZYME® IMGI, and G-ZYME® G993, KETOMAX®, G-ZYME® G993, G-ZYME® G993 liquid, and GENSWEET® IGI. Following isomerization, the mixture typically contains about 40-45% fructose, *e.g.*, 42% fructose.

### 4.5. Fermentation

The soluble starch hydrolysate, particularly a glucose rich syrup, can be fermented by contacting the starch hydrolysate with a fermenting organism typically at a temperature around 32°C, such as from 30°C to 35°C for alcohol-producing yeast. The temperature and pH of the fermentation will depend upon the fermenting organism. EOF products include metabolites, such as citric acid, lactic acid, succinic acid, monosodium glutamate, gluconic acid, sodium gluconate, calcium gluconate, potassium gluconate, itaconic acid and other carboxylic acids, glucono delta-lactone, sodium erythorbate, lysine and other amino acids, omega 3 fatty acid, butanol, isoprene, 1,3-propanediol and other biomaterials.

Ethanologenic microorganisms include yeast, such as Saccharomyces cerevisiae and bacteria, *e.g.*, *Zymomonas moblis*, expressing alcohol dehydrogenase and pyruvate decarboxylase. The ethanologenic microorganism can express xylose reductase and xylitol dehydrogenase, which convert xylose to xylulose. Improved strains of ethanologenic microorganisms, which can withstand higher temperatures, for example, are known in the art and can be used. See Liu et al. (2011) Sheng Wu Gong Cheng Xue Bao 27: 1049-56. Commercial sources of yeast include ETHANOL RED® (LeSaffre); FERMAX™ (Martrex), THERMOSACC® (Lallemand); RED STAR® (Red Star); FERMIOL® (DSM Specialties); and SUPERSTART® (Alltech). Microorganisms that produce other metabolites, such as citric acid and lactic acid, by fermentation are also known in the art. See, *e.g.*, Papagianni (2007) Biotechnol. Adv. 25: 244-63; John et al. (2009) Biotechnol. Adv. 27: 145-52.

The saccharification and fermentation processes may be carried out as an SSF process. Fermentation may comprise subsequent enrichment, purification, and recovery of ethanol, for example. During the fermentation, the ethanol content of the broth or "beer" may reach about 8-18% v/v, *e.g.*, 14-15% v/v. The broth may be distilled to produce enriched, *e.g.*, 96% pure, solutions of ethanol. Further, $CO_2$ generated by fermentation may be collected with a $CO_2$ scrubber, compressed, and marketed for other uses, *e.g.*, carbonating beverage or dry ice production. Solid waste from the fermentation process may be used as protein-rich products, *e.g.*, livestock feed.

As mentioned above, an SSF process can be conducted with fungal cells that express and secrete amylase continuously throughout SSF. The fungal cells expressing amylase also can be the fermenting microorganism, *e.g.*, an ethanologenic microorganism. Ethanol production thus can be carried out using a fungal cell that expresses sufficient amylase so that less or no enzyme has to be added exogenously. The fungal host cell can be from an appropriately engineered fungal strain. Fungal host cells that express and secrete other enzymes, in addition to amylase, also can be used. Such cells may express glucoamylase and/or a pullulanase, phytase, alpha-glucosidase, isoamylase, beta-amylase cellulase, xylanase, other hemicellulases, protease, beta-glucosidase, pectinase, esterase, redox enzymes, transferase, or other enzyme.

A variation on this process is a "fed-batch fermentation" system, where the substrate is added in increments as the fermentation progresses. Fed-batch systems are useful when catabolite repression may inhibit the metabolism of the cells and where it is desirable to have limited amounts of substrate in the medium. The actual substrate concentration in fed-batch systems is estimated by the changes of measurable factors such as pH, dissolved oxygen and the partial pressure of waste gases, such as $CO_2$. Batch and fed-batch fermentations are common and well known in the art.

Continuous fermentation is an open system where a defined fermentation medium is added continuously to a bioreactor, and an equal amount of conditioned medium is removed simultaneously for processing. Continuous fermentation generally maintains the cultures at a constant high density where cells are primarily in log phase growth. Continuous fermentation permits modulation of cell growth and/or product concentration. For example, a limiting nutrient such as the carbon source or nitrogen source is maintained at a fixed rate and all other parameters are allowed to moderate. Because growth is maintained at a steady state, cell loss due to medium being drawn off should be balanced against the cell growth rate in the fermentation. Methods of optimizing continuous fermentation processes and maximizing the rate of product formation are well known in the art of industrial microbiology.

### 4.6. Compositions Comprising Variants Amylases

Variant amylases may be combined with a glucoamylase (EC 3.2.1.3), *e.g.*, a *Trichoderma* glucoamylase or variant thereof. An exemplary glucoamylase is *Trichoderma reesei* glucoamylase (TrGA) and variants thereof that possess superior specific activity and thermal stability. See U.S. Published Applications Nos. 2006/0094080, 2007/0004018, and 2007/0015266 (Danisco US Inc.). Suitable variants of TrGA include those with glucoamylase activity and at least 80%, at least 90%, or at least 95% sequence identity to wild-type TrGA. Variant amylases advantageously increase the yield of glucose produced in a saccharification process catalyzed by TrGA.

Alternatively, the glucoamylase may be another glucoamylase derived from plants (including algae), fungi, or bacteria. For example, the glucoamylases may be *Aspergillus niger* G1 or G2 glucoamylase or its variants (*e.g.*, Boel et al. (1984) EMBO J. 3: 1097-1102; WO 92/00381; WO 00/04136 (Novo Nordisk A/S)); and *A. awamori* glucoamylase (*e.g.*, WO 84/02921 (Cetus Corp.)). Other contemplated *Aspergillus* glucoamylase include variants with enhanced thermal stability, *e.g.*, G137A and G139A (Chen et al. (1996) Prot. Eng. 9: 499-505); D257E and D293E/Q (Chen et al. (1995) Prot. Eng. 8: 575-582); N182 (Chen et al. (1994) Biochem. J. 301: 275-281); A246C (Fierobe et al. (1996) Biochemistry 35: 8698-8704); and variants with Pro residues in positions A435 and S436 (Li et al. (1997) Protein Eng. 10: 1199-1204). Other contemplated glucoamylases include *Talaromyces* glucoamylases, in particular derived from *T. emersonii* (*e.g.*, WO 99/28448 (Novo Nordisk A/S), *T. leycettanus* (*e.g.*, U.S. Patent No. RE 32,153 (CPC International, Inc.)), *T. duponti*, or *T. thermophilus* (*e.g.*, U.S. Patent No. 4,587,215). Contemplated bacterial glucoamylases include glucoamylases from the genus *Clostridium*, in particular *C. thermoamylolyticum* (*e.g.*, EP 135,138 (CPC International, Inc.) and *C. thermohydrosulfuricum* (*e.g.*, WO 86/01831 (Michigan Biotechnology Institute)). Suitable glucoamylases include the glucoamylases derived from *Aspergillus oryzae*, such as a glucoamylase in WO 00/04136 (Novo Nordisk A/S). Also suitable are commercial glucoamylases, such as AMG 200L; AMG 300 L; SAN™ SUPER and AMG™ E (Novozymes); OPTIDEX® 300 and OPTIDEX L-400 (Danisco US Inc.); AMIGASE™ and AMIGASE™ PLUS (DSM); G-ZYME® G900 (Enzyme Bio-Systems); and G-ZYME® G990 ZR (*A. niger* glucoamylase with a low protease content). Still other suitable glucoamylases include *Aspergillus fumigatus* glucoamylase, *Talaromyces glucoamylase, Thielavia* glucoamylase, *Trametes* glucoamylase, *Thermomyces* glucoamylase, *Athelia* glucoamylase, *Humicola* glucoamylase (*e.g.*, HgGA), *Penicillium* glucoamylase, *Artomyces* glucoamylase, *Gloeophyllum* glucoamylase, *Pycnoporus* glucoamylase, or *Steccherinum* glucoamylase. Glucoamylases typically are added in an amount of about 0.1 - 2 glucoamylase units (GAU)/g ds, *e.g.*, about 0.16 GAU/g ds, 0.23 GAU/g ds, or 0.33 GAU/g ds.

Other suitable enzymes that can be used with amylase include a phytase, protease, pullulanase, β-amylase, isoamylase, a different α-amylase, alpha-glucosidase, cellulase, xylanase, other hemicellulases, beta-glucosidase, transferase, pectinase, lipase, cutinase, esterase, redox enzymes, or a combination thereof. For example, a debranching enzyme, such as an isoamylase (EC 3.2.1.68), may be added in effective amounts well known to the person skilled in the art. A pullulanase (EC 3.2.1.41), *e.g.*, PROMOZYME®, is also suitable. Pullulanase typically is added at 100 U/kg ds. Further suitable enzymes include proteases, such as fungal and bacterial proteases. Fungal proteases include those obtained from *Aspergillus,* such as *A. niger*, *A. awamori*, *A. oryzae*; *Mucor* (*e.g.*, *M. miehei*); *Rhizopus*; and *Trichoderma.*

β-Amylases (EC 3.2.1.2) are exo-acting maltogenic amylases, which catalyze the hydrolysis of 1,4-α-glucosidic linkages in amylopectin and related glucose polymers, thereby releasing maltose. β-Amylases have been isolated from various plants and microorganisms. See Fogarty et al. (1979) in Progress in Industrial Microbiology, Vol. 15, pp. 112-115. These β-Amylases have optimum temperatures in the range from 40°C to 65°C and optimum pH in the range from about 4.5 to about 7.0. Contemplated β-amylases include, but are not limited to, β-amylases from barley SPEZYME® BBA 1500, SPEZYME® DBA, OPTIMALT™ ME, OPTIMALT™ BBA (Danisco US Inc.); and NOVOZYM™ WBA (Novozymes A/S).

Compositions comprising the present amylases may be aqueous or non-aqueous formulations, granules, powders, gels, slurries, pastes, etc., which may further comprise any one or more of the additional enzymes listed, herein, along with buffers, salts, preservatives, water, co-solvents, surfactants, and the like. Such compositions may work in combination with endogenous enzymes or other ingredients already present in a slurry, water bath, washing machine, food or drink product, etc., for example, endogenous plant (including algal) enzymes, residual enzymes from a prior processing step, and the like.

## 5. Compositions and Methods for Baking and Food Preparation

Also described is a "food composition," including but not limited to a food product, animal feed and/or food/feed additives, comprising an amylase defined in the claims, and methods for preparing such a food composition comprising mixing variant amylase with one or more food ingredients, or uses thereof.

Furthermore, also described is the use of an amylase in the preparation of a food composition, wherein the food composition is baked subsequent to the addition of the claimed polypeptide. As used herein the term "baking composition" means any composition and/or additive prepared in the process of providing a baked food product, including but not limited to baker's flour, a dough, a baking additive and/or a baked product. The food composition or additive may be liquid or solid.

## 6. Textile Desizing Compositions and Use

Also described are compositions and methods of treating fabrics (*e.g.*, to desize a textile) using an amylase defined in the claims. Fabric-treating methods are well known in the art (see, *e.g.*, U.S. Patent No. 6,077,316). For example, the feel and appearance of a fabric can be improved by a method comprising contacting the fabric with an amylase in a solution. The fabric can be treated with the solution under pressure.

An amylase as defined in the claims can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. The amylase can be applied during or after the weaving to remove these sizing starch or starch derivatives. After weaving, the amylase can be used to remove the size coating before further processing the fabric to ensure a homogeneous and wash-proof result.

The amylase can be used alone or with other desizing chemical reagents and/or desizing enzymes to desize fabrics, including cotton-containing fabrics, as detergent additives, *e.g.*, in aqueous compositions. The amylase may also be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes to provide softness to the fabric and make the cotton more accessible to the subsequent enzymatic finishing steps. The amylase can be used in methods of finishing denim garments (*e.g.*, a "bio-stoning process"), enzymatic desizing and providing softness to fabrics, and/or finishing process.

## 7. Cleaning Compositions

An aspect of the present compositions and methods is a cleaning composition that includes an amylase defined in the claims as a component. The amylase polypeptide can be used as a component in detergent compositions for, *e.g.*, hand washing, laundry washing, dishwashing, and other hard-surface cleaning. Such compositions include heavy duty liquid (HDL), heavy duty dry (HDD), and hand (manual) laundry detergent compositions, including unit dose format laundry detergent compositions, and automatic dishwashing (ADW) and hand (manual) dishwashing compositions, including unit dose format dishwashing compositions.

### 7.1. Overview

Preferably, an amylase as defined in the claims is incorporated into detergents at or near a concentration conventionally used for amylase in detergents. For example, the amylase polypeptide may be added in amount corresponding to 0.00001 - 1 mg (calculated as pure enzyme protein) of amylase per liter of wash/dishwash liquor. Exemplary formulations are provided herein, as exemplified by the following:

An amylase polypeptide as defined in the claims may be a component of a detergent composition, as the only enzyme or with other enzymes including other amylolytic enzymes. As such, it may be included in the detergent composition in the form of a non-dusting granulate, a stabilized liquid, or a protected enzyme. Non-dusting granulates may be produced, *e.g.*, as disclosed in U.S. Patent Nos. 4,106,991 and 4,661,452 and may optionally be coated by methods known in the art. Examples of waxy coating materials are poly(ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Other enzyme stabilizers are known in the art. Protected enzymes may be prepared according to the method disclosed in for example EP 238 216. Polyols have long been recognized as stabilizers of proteins, as well as improving protein solubility.

The detergent composition may be in any useful form, *e.g.*, as powders, granules, pastes, bars, or liquid. A liquid detergent may be aqueous, typically containing up to about 70% of water and 0% to about 30% of organic solvent. It may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); α-olefinsulfonate (AOS); alkyl sulfate (fatty alcohol sulfate) (AS); alcohol ethoxysulfate (AEOS or AES); secondary alkanesulfonates (SAS); α-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as proteases, another amylolytic enzyme, cutinase, lipase, cellulase, pectate lyase, perhydrolase, xylanase, peroxidase, and/or laccase in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (*e.g.*, SKS-6 from Hoechst). The detergent may also be unbuilt, *i.e.* essentially free of detergent builder. The enzymes can be used in any composition compatible with the stability of the enzyme. Enzymes generally can be protected against deleterious components by known forms of encapsulation, for example, by granulation or sequestration in hydro gels. Enzymes, and specifically amylases, either with or without starch binding domains, can be used in a variety of compositions including laundry and dishwashing applications, surface cleaners, as well as in compositions for ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a $H_2O_2$ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxyacids (*e.g.*, the amide, imide, or sulfone type peroxyacids). The bleaching system can also be an enzymatic bleaching system, for example, perhydrolase, such as that described in International PCT Application WO 2005/056783.

The enzymes of the detergent composition may be stabilized using conventional stabilizing agents, *e.g.*, a polyol such as propylene glycol or glycerol; a sugar or sugar alcohol; lactic acid; boric acid or a boric acid derivative such as, *e.g.*, an aromatic borate ester; and the composition may be formulated as described in, *e.g.*, WO 92/19709 and WO 92/19708.

The detergent may also contain other conventional detergent ingredients such as *e.g.*, fabric conditioners including clays, foam boosters, suds suppressors, anti-corrosion agents, soil-suspending agents, anti-soil redeposition agents, dyes, bactericides, tarnish inhibiters, optical brighteners, or perfumes.

The pH (measured in aqueous solution at use concentration) is usually neutral or alkaline, *e.g.*, pH about 7.0 to about 11.0.

Particular forms of detergent compositions for inclusion of the present $\alpha$-amylase are described, below. Many of these composition can be provided in unit dose format for ease of use. Unit dose formulations and packaging are described in, for example, US20090209445A1, US20100081598A1, US7001878B2, EP1504994B1, WO2001085888A2, WO2003089562A1, WO2009098659A1, WO2009098660A1, WO2009112992A1, WO2009124160A1, WO2009152031A1, WO2010059483A1, WO2010088112A1, WO2010090915A1, WO2010135238A1, WO2011094687A1, WO2011094690A1, WO2011127102A1, WO2011163428A1, WO2008000567A1, WO2006045391A1, WO2006007911A1, WO2012027404A1, EP1740690B1, WO2012059336A1, US6730646B1, WO2008087426A1, WO2010116139A1, and WO2012104613A1.

**7.2. Heavy Duty Liquid (HDL) laundry detergent composition**

Exemplary HDL laundry detergent compositions includes a detersive surfactant (10%-40% wt/wt), including an anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof), and optionally non-ionic surfactant (selected from a group of linear or branched or random chain, substituted or unsubstituted alkyl alkoxylated alcohol, for example a C8-C18 alkyl ethoxylated alcohol and/or C6-C12 alkyl phenol alkoxylates), wherein the weight ratio of anionic detersive surfactant (with a hydrophilic index (HIc) of from 6.0 to 9) to non-ionic detersive surfactant is greater than 1: 1. Suitable detersive surfactants also include cationic detersive surfactants (selected from a group of alkyl pyridinium compounds, alkyl quarterny ammonium compounds, alkyl quarterny phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition may optionally include, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C1-C6 carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C4-C25 alkyl group, polypropylene, polybutylene, vinyl ester of a saturated C1-C6 mono-carboxylic acid, C1-C6 alkyl ester of acrylic or methacrylic acid, and mixtures thereof.

The composition may include additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), antiredeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition may further include saturated or unsaturated fatty acid, preferably saturated or unsaturated C12-C24 fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition may further include dye transfer inhibiting agents, examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents, examples of which include ethylene-diamine-tetraacetic acid (EDTA), diethylene triamine penta methylene phosphonic acid (DTPMP), hydroxy-ethane diphosphonic acid (HEDP), ethylenediamine N,N'-disuccinic acid (EDDS), methyl glycine diacetic acid (MGDA), diethylene triamine penta acetic acid (DTPA), propylene diamine tetracetic acid (PDTA), 2-hydroxypyridine-N-oxide (HPNO), or methyl glycine diacetic acid (MGDA), glutamic acid N,N-diacetic acid (N,N-dicarboxymethyl glutamic acid tetrasodium salt (GLDA), nitrilotriacetic acid (NTA), 4,5-dihydroxy-m-benzenedisulfonic acid, citric acid and any salts thereof, N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition preferably included enzymes (generally about 0.01 wt% active enzyme to 0.03wt% active enzyme) selected from proteases, amylases, lipases, cellulases, choline oxidases, peroxidases/oxidases, pectate lyases, mannanases, cutinases, laccases, phospholipases, lysophospholipases, acyltransferases, perhydrolases, arylesterases, and any mixture thereof. The composition may include an enzyme stabilizer (examples of which include polyols such as propylene glycol or glycerol, sugar or sugar alcohol, lactic acid, reversible protease inhibitor, boric acid, or a boric acid derivative, *e.g.*, an aromatic borate ester, or a phenyl boronic acid derivative such as 4-formylphenyl boronic acid).

The composition optionally includes silicone or fatty-acid based suds suppressors; hueing dyes, calcium and magnesium cations, visual signaling ingredients, anti-foam (0.001 wt% to about 4.0wt%), and/or structurant/thickener (0.01 wt% to 5 wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

The composition can be any liquid form, for example a liquid or gel form, or any combination thereof. The composition may be in any unit dose form, for example a pouch.

## 7.3. Heavy Duty Dry/Solid (HDD) laundry detergent composition

Exemplary HDD laundry detergent compositions includes a detersive surfactant, including anionic detersive surfactants (*e.g.*, linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (*e.g.*, linear or branched or random chain, substituted or unsubstituted C8-C18 alkyl ethoxylates, and/or C6-C12 alkyl phenol alkoxylates), cationic detersive surfactants (*e.g.*, alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (*e.g.*, alkanolamine sulpho-betaines), ampholytic surfactants, semi-polar non-ionic surfactants, and mixtures thereof; builders including phosphate free builders (for example zeolite builders examples which include zeolite A, zeolite X, zeolite P and zeolite MAP in the range of 0 wt% to less than 10 wt%), phosphate builders (for example sodium tri-polyphosphate in the range of 0 wt% to less than 10 wt%), citric acid, citrate salts and nitrilotriacetic acid, silicate salt (*e.g.*, sodium or potassium silicate or sodium meta-silicate in the range of 0 wt% to less than 10 wt%, or layered silicate (SKS-6)); carbonate salt (*e.g.*, sodium carbonate and/or sodium bicarbonate in the range of 0 wt% to less than 80 wt%); and bleaching agents including photobleaches (*e.g.*, sulfonated zinc phthalocyanines, sulfonated aluminum phthalocyanines, xanthenes dyes, and mixtures thereof) hydrophobic or hydrophilic bleach activators (*e.g.*, dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, nonanoyloxybenzene sulfonate-NOBS, nitrile quats, and mixtures thereof), sources of hydrogen peroxide (*e.g.*, inorganic perhydrate salts examples of which include mono or tetra hydrate sodium salt of perborate, percarbonate, persulfate, perphosphate, or persilicate), preformed hydrophilic and/or hydrophobic peracids (*e.g.*, percarboxylic acids and salts, percarbonic acids and salts, perimidic acids and salts, peroxymonosulfuric acids and salts, and mixtures thereof), and/or bleach catalysts (*e.g.*, imine bleach boosters (examples of which include iminium cations and polyions), iminium zwitterions, modified amines, modified amine oxides, N-sulphonyl imines, N-phosphonyl imines, N-acyl imines, thiadiazole dioxides, perfluoroimines, cyclic sugar ketones, and mixtures thereof, and metal-containing bleach catalysts (*e.g.*, copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid), and watersoluble salts thereof).

The composition preferably includes enzymes, *e.g.*, proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and any mixture thereof.

The composition may optionally include additional detergent ingredients including perfume microcapsules, starch encapsulated perfume accord, hueing agents, additional polymers, including fabric integrity and cationic polymers, dye-lock ingredients, fabric-softening agents, brighteners (for example C.I. Fluorescent brighteners), flocculating agents, chelating agents, alkoxylated polyamines, fabric deposition aids, and/or cyclodextrin.

### 7.4. Automatic dishwashing (ADW) detergent composition

As noted, above, the present $\alpha$-amylase variants are particularly effective in ADW applications. Exemplary ADW detergent composition includes non-ionic surfactants, including ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% including phosphate builders (*e.g.*, mono-phosphates, di-phosphates, tri-polyphosphates, other oligomeric-poylphosphates, sodium tripolyphosphate-STPP) and phosphate-free builders (*e.g.*, amino acid-based compounds including methylglycine-diacetic acid (MGDA) and salts and derivatives thereof, glutamic-N,N-diacetic acid (GLDA) and salts and derivatives thereof, iminodisuccinic acid (IDS) and salts and derivatives thereof, carboxy methyl inulin and salts and derivatives thereof, nitrilotriacetic acid (NTA), diethylene triamine penta acetic acid (DTPA), B-alaninediacetic acid (B-ADA) and their salts, homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers in the range of about 0.1 % to about 50% by weight to provide dimensional stability; drying aids in the range of about 0.1 % to about 10% by weight (*e.g.*, polyesters, especially anionic polyesters, optionally together with further monomers with 3 to 6 functionalities - typically acid, alcohol or ester functionalities which are conducive to polycondensation, polycarbonate-, polyurethane- and/or polyurea-polyorganosiloxane compounds or precursor compounds, thereof, particularly of the reactive cyclic carbonate and urea type); silicates in the range from about 1 % to about 20% by weight (including sodium or potassium silicates for example sodium disilicate, sodium meta-silicate and crystalline phyllosilicates); inorganic bleach (*e.g.*, perhydrate salts such as perborate, percarbonate, perphosphate, persulfate and persilicate salts) and organic bleach (*e.g.*, organic peroxyacids, including diacyl and tetraacylperoxides, especially diperoxydodecanedioc acid, diperoxytetradecanedioc acid, and diperoxyhexadecanedioc acid); bleach activators (*i.e.*, organic peracid precursors in the range from about 0.1 % to about 10% by weight); bleach catalysts (*e.g.*, manganese triazacyclononane and related complexes, Co, Cu, Mn, and Fe bispyridylamine and related complexes, and pentamine acetate cobalt(III) and related complexes); metal care agents in the range from about 0.1% to 5% by weight (*e.g.*, benzatriazoles, metal salts and complexes, and/or silicates); enzymes in the range from about 0.01 to 5.0 mg of active enzyme per gram of automatic dishwashing detergent composition (*e.g.*, proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and mixtures thereof); and enzyme stabilizer components (*e.g.*, oligosaccharides, polysaccharides, and inorganic divalent metal salts).

### 7.5. Additional detergent compositions

Additional exemplary detergent formulations to which the present amylase can be added are described, below, in the numbered paragraphs.

1) A detergent composition formulated as a granulate having a bulk density of at least 600 g/L comprising linear alkylbenzenesulfonate (calculated as acid) about 7% to about 12%; alcohol ethoxysulfate (*e.g.*, C12-18 alcohol, 1-2 ethylene oxide (EO)) or alkyl sulfate (*e.g.*, C16-18) about 1% to about 4%; alcohol ethoxylate (*e.g.*, C14-15 alcohol, 7 EO) about 5% to about 9%; sodium carbonate about 14% to about 20%; soluble silicate about 2 to about 6%; zeolite about 15% to about 22%; sodium sulfate 0% to about 6%; sodium citrate/citric acid about 0% to about 15%; sodium perborate about 11% to about 18%; TAED about 2% to about 6%; carboxymethylcellulose (CMC) and 0% to about 2%; polymers (*e.g.*, maleic/acrylic acid, copolymer, PVP, PEG) 0-3%; enzymes (calculated as pure enzyme) 0.0001-0.1% protein; and minor ingredients (*e.g.*, suds suppressors, perfumes, optical brightener, photobleach) 0-5%.

2) A detergent composition formulated as a granulate having a bulk density of at least 600 g/L comprising linear alkylbenzenesulfonate (calculated as acid) about 6% to about 11%; alcohol ethoxysulfate (*e.g.*, C12-18 alcohol, 1-2 EO) or alkyl sulfate *(e.g.,* C16-18) about 1% to about 3%; alcohol ethoxylate (*e.g.*, C14-15 alcohol, 7 EO) about 5% to about 9%; sodium carbonate about 15% to about 21%; soluble silicate about 1% to about 4%; zeolite about 24% to about 34%; sodium sulfate (*e.g.*, $Na_2SO_4$) about 4% to about 10%; sodium citrate/citric acid 0% to about 15%; carboxymethylcellulose (CMC) 0% to about 2%; polymers (*e.g.*, maleic/acrylic acid copolymer, PVP, PEG) 1-6%; enzymes (calculated as pure enzyme protein) 0.0001-0.1%; minor ingredients (*e.g.*, suds suppressors, perfume) 0-5%.

3) A detergent composition formulated as a granulate having a bulk density of at least 600 g/L comprising linear alkylbenzenesulfonate (calculated as acid) about 5% to about 9%; alcohol ethoxylate (*e.g.*, C12-15 alcohol, 7 EO) about 7% to about 14%; Soap as fatty acid (*e.g.*, C16-22 fatty acid) about 1 to about 3%; sodium carbonate about 10% to about 17%; soluble silicate about 3% to about 9%; zeolite about 23% to about 33%; sodium sulfate 0% to about 4%; sodium perborate about 8% to about 16%; TAED about 2% to about 8%; phosphonate (*e.g.*, EDTMPA) 0% to about 1%; carboxymethylcellulose (CMC) 0% to about 2%; polymers (*e.g.*, maleic/acrylic acid copolymer, PVP, PEG) 0-3%; enzymes (calculated as pure enzyme protein) 0.0001-0.1%; minor ingredients (e.g., suds suppressors, perfume, optical brightener) 0-5%.

4) A detergent composition formulated as a granulate having a bulk density of at least 600 g/L comprising linear alkylbenzenesulfonate (calculated as acid) about 8% to about 12%; alcohol ethoxylate (*e.g.*, C12-15 alcohol, 7 EO) about 10% to about 25%; sodium carbonate about 14% to about 22%; soluble silicate about 1% to about 5%; zeolite about 25% to about 35%; sodium sulfate 0% to about 10%; carboxymethylcellulose (CMC) 0% to about 2%; polymers (*e.g.*, maleic/acrylic acid copolymer, PVP, PEG) 1-3%; enzymes (calculated as pure enzyme protein) 0.0001-0.1%; and minor ingredients (*e.g.*, suds suppressors, perfume) 0-5%.

5) An aqueous liquid detergent composition comprising linear alkylbenzenesulfonate (calculated as acid) about 15% to about 21%; alcohol ethoxylate (*e.g.*, C12-15 alcohol, 7 EO or C12-15 alcohol, 5 EO) about 12% to about 18%; soap as fatty acid (*e.g.*, oleic acid) about 3% to about 13%; alkenylsuccinic acid (C12-14) 0% to about 13%; aminoethanol about 8% to about 18%; citric acid about 2% to about 8%; phosphonate 0% to about 3%; polymers (*e.g.*, PVP, PEG) 0% to about 3%; borate 0% to about 2%; ethanol 0% to about 3%; propylene glycol about 8% to about 14%; enzymes (calculated as pure enzyme protein) 0.0001-0.1%; and minor ingredients (*e.g.*, dispersants, suds suppressors, perfume, optical brightener) 0-5%.

6) An aqueous structured liquid detergent composition comprising linear alkylbenzenesulfonate (calculated as acid) about 15% to about 21%; alcohol ethoxylate (*e.g.*, C12-15 alcohol, 7 EO, or C12-15 alcohol, 5 EO) 3-9%; soap as fatty acid (*e.g.*, oleic acid) about 3% to about 10%; zeolite about 14% to about 22%; potassium citrate about 9% to about 18%; borate 0% to about 2%; carboxymethylcellulose (CMC) 0% to about 2%; polymers (*e.g.*, PEG, PVP) 0% to about 3%; anchoring polymers such as, *e.g.*, lauryl methacrylate/acrylic acid copolymer; molar ratio 25: 1, MW 3800) 0% to about 3%; glycerol 0% to about 5%; enzymes (calculated as pure enzyme protein) 0.0001-0.1%; and minor ingredients (*e.g.*, dispersants, suds suppressors, perfume, optical brighteners) 0-5%.

7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate about 5% to about 10%; Soluble silicate about 1% to about 4%; zeolite about 20% to about 40%; Sodium sulfate about 2% to about 8%; sodium perborate about 12% to about 18%; TAED about 2% to about 7%; polymers (*e.g.*, maleic/acrylic acid copolymer, PEG) about 1% to about 5%; enzymes (calculated as pure enzyme protein) 0.0001-0.1%; and minor ingredients (*e.g.*, optical brightener, suds suppressors, perfume) 0-5%.

8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate about 4% to about 10%; soluble silicate about 1% to about 4%; zeolite about 30% to about 50%; sodium sulfate about 3% to about 11%; sodium citrate about 5% to about 12%; polymers (*e.g.*, PVP, maleic/acrylic acid copolymer, PEG) about 1% to about 5%; enzymes (calculated as pure enzyme protein) 0.0001-0.1%; and minor ingredients (*e.g.*, suds suppressors, perfume) 0-5%.

9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate about 14% to about 22%; zeolite about 18% to about 32%; sodium sulfate about 5% to about 20%; sodium citrate about 3% to about 8%; sodium perborate about 4% to about 9%; bleach activator (*e.g.*, NOBS or TAED) about 1% to about 5%; carboxymethylcellulose (CMC) 0% to about 2%; polymers (*e.g.*, polycarboxylate or PEG) about 1% to about 5%; enzymes (calculated as pure enzyme protein) 0.0001-0.1%; and minor ingredients (*e.g.*, optical brightener, perfume) 0-5%.

10) An aqueous liquid detergent composition comprising linear alkylbenzenesulfonate (calculated as acid) about 15% to about 23%; alcohol ethoxysulfate (*e.g.*, C12-15 alcohol, 2-3 EO) about 8% to about 15%; alcohol ethoxylate (*e.g.*, C12-15 alcohol, 7 EO, or C12-15 alcohol, 5 EO) about 3% to about 9%; soap as fatty acid (*e.g.*, lauric acid) 0% to about 3%; aminoethanol about 1% to about 5%; sodium citrate about 5% to about 10%; hydrotrope (*e.g.*, sodium toluensulfonate) about 2% to about 6%; borate 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e.g.*, polymers, dispersants, perfume, optical brighteners) 0-5%.

11) An aqueous liquid detergent composition comprising linear alkylbenzenesulfonate (calculated as acid) about 20% to about 32%; alcohol ethoxylate (*e.g.*, C12-15 alcohol, 7 EO, or C12-15 alcohol, 5 EO) 6-12%; aminoethanol about 2% to about 6%; citric acid about 8% to about 14%; borate about 1% to about 3%; polymer (*e.g.*, maleic/acrylic acid copolymer, anchoring polymer such as, *e.g.*, lauryl methacrylate/acrylic acid copolymer) 0% to about 3%; glycerol about 3% to about 8%; enzymes (calculated as pure enzyme protein) 0.0001-0.1%; and minor ingredients (*e.g.*, hydrotropes, dispersants, perfume, optical brighteners) 0-5%.

12) A detergent composition formulated as a granulate having a bulk density of at least 600 g/L comprising anionic surfactant (linear alkylbenzenesulfonate, alkyl sulfate, α-olefinsulfonate, α-sulfo fatty acid methyl esters, alkanesulfonates, soap) about 25% to about 40%; nonionic surfactant (*e.g.*, alcohol ethoxylate) about 1% to about 10%; sodium carbonate about 8% to about 25%; soluble silicates about 5% to about 15%; sodium sulfate 0% to about 5%; zeolite about 15% to about 28%; sodium perborate 0% to about 20%; bleach activator (TAED or NOBS) about 0% to about 5%; enzymes (calculated as pure enzyme protein) 0.0001-0.1%; minor ingredients (*e.g.*, perfume, optical brighteners) 0-3%.

13) Detergent compositions as described in compositions 1)-12) supra, wherein all or part of the linear alkylbenzenesulfonate is replaced by (C12-C18) alkyl sulfate.

14) A detergent composition formulated as a granulate having a bulk density of at least 600 g/L comprising (C12-C18) alkyl sulfate about 9% to about 15%; alcohol ethoxylate about 3% to about 6%; polyhydroxy alkyl fatty acid amide about 1% to about 5%; zeolite about 10% to about 20%; layered disilicate (*e.g.*, SK56 from Hoechst) about 10% to about 20%; sodium carbonate about 3% to about 12%; soluble silicate 0% to about 6%; sodium citrate about 4% to about 8%; sodium percarbonate about 13% to about 22%; TAED about 3% to about 8%; polymers (*e.g.*, polycarboxylates and PVP) 0% to about 5%; enzymes (calculated as pure enzyme protein) 0.0001-0.1%; and minor ingredients (*e.g.*, optical brightener, photobleach, perfume, suds suppressors) 0-5%.

15) A detergent composition formulated as a granulate having a bulk density of at least 600 g/L comprising (C12-C18) alkyl sulfate about 4% to about 8%; alcohol ethoxylate about 11% to about 15%; soap about 1% to about 4%; zeolite MAP or zeolite A about 35% to about 45%; sodium carbonate about 2% to about 8%; soluble silicate 0% to about 4%; sodium percarbonate about 13% to about 22%; TAED 1-8%; carboxymethylcellulose (CMC) 0% to about 3%; polymers (*e.g.*, polycarboxylates and PVP) 0% to about 3%; enzymes (calculated as pure enzyme protein) 0.0001-0.1%; and minor ingredients (*e.g.*, optical brightener, phosphonate, perfume) 0-3%.

16) Detergent formulations as described in 1)-15) supra, which contain a stabilized or encapsulated peracid, either as an additional component or as a substitute for already specified bleach systems.

17) Detergent compositions as described supra in 1), 3), 7), 9), and 12), wherein perborate is replaced by percarbonate.

18) Detergent compositions as described supra in 1), 3), 7), 9), 12), 14), and 15), which additionally contain a manganese catalyst. The manganese catalyst for example is one of the compounds described by Hage et al. ((1994) Nature 369: 637-639).

19) Detergent composition formulated as a non-aqueous detergent liquid comprising a liquid nonionic surfactant such as, *e.g.,* linear alkoxylated primary alcohol, a builder system (*e.g.*, phosphate), an enzyme(s), and alkali. The detergent may also comprise anionic surfactant and/or a bleach system.

As above, the present amylase polypeptide may be incorporated at a concentration conventionally employed in detergents. It is at present contemplated that, in the detergent composition, the enzyme may be added in an amount corresponding to 0.00001-1.0 mg (calculated as pure enzyme protein) of amylase polypeptide per liter of wash liquor.

The detergent composition may also contain other conventional detergent ingredients, *e.g.*, deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

The detergent composition may be formulated as a hand (manual) or machine (automatic) laundry detergent composition, including a laundry additive composition suitable for pre-treatment of stained fabrics and a rinse added fabric softener composition, or be formulated as a detergent composition for use in general household hard surface cleaning operations, or be formulated for manual or automatic dishwashing operations.

Any of the cleaning compositions described, herein, may include any number of additional enzymes. In general, the enzyme(s) should be compatible with the selected detergent, (*e.g.*, with respect to pH-optimum, compatibility with other enzymatic and non-enzymatic ingredients, and the like), and the enzyme(s) should be present in effective amounts. The following enzymes are provided as examples.

Proteases: Suitable proteases include those of animal, vegetable or microbial origin. Chemically modified or protein engineered mutants are included, as well as naturally processed proteins. The protease may be a serine protease or a metalloprotease, an alkaline microbial protease, a trypsin-like protease, or a chymotrypsin-like protease. Examples of alkaline proteases are subtilisins, especially those derived from *Bacillus*, *e.g.*, subtilisin Novo, subtilisin Carlsberg, subtilisin 309, subtilisin 147, and subtilisin 168 (see, *e.g.*, WO 89/06279). Exemplary proteases include but are not limited to those described in WO95/23221, WO 92/21760, WO2008010925, WO20100566356, WO2011072099, WO201113022, WO2011140364, WO 2012151534, WO2015038792, WO2015089441, WO2015089447, WO2015143360, WO2016001449, WO2016001450, WO2016061438, WO2016069544,WO2016069548, WO2016069552, WO 2016069557, WO2016069563, WO2016069569, WO2016087617, WO2016087619, WO2016145428, WO2016174234, WO2016183509, WO2016202835, WO2016205755, US 2008/0090747, US 5,801,039, US 5,340,735, US 5,500,364, US 5,855,625, RE 34,606, US 5,955,340, US 5,700,676, US 6,312,936, US 6,482,628, US8530219, US Provisional Appl Nos. 62/331282, 62/343618, 62/351649, 62/437171, 62/437174, and 62/437509, and PCT Appl Nos. PCT/CN2017/076749 and, as well as metalloproteases described in WO 2007/044993, WO 2009/058303, WO 2009/058661, WO 2014/071410, WO 2014/194032, WO 2014/194034, WO 2014/194054, and WO 2014/194117.

Exemplary commercial proteases include, but are not limited to MAXATASE, MAXACAL, MAXAPEM, OPTICLEAN®, OPTIMASE®, PROPERASE®, PURAFECT®, PURAFECT® OXP, PURAMAX®, EXCELLASE®, PREFERENZ™ proteases (e.g. P100, P110, P280), EFFECTENZ™ proteases (e.g. P1000, P1050, P2000), EXCELLENZ™ proteases (e.g. P1000), ULTIMASE®, and PURAFAST (Danisco US); ALCALASE®, ALCALASE® ULTRA, BLAZE®, BLAZE® EVITY®, BLAZE® EVITY® 16L, CORONASE®, SAVINASE®, SAVINASE® ULTRA, SAVINASE® EVITY®, SAVINASE® EVERIS®, PRIMASE, DURAZYM, POLARZYME®, OVOZYME®, KANNASE®, LIQUANASE®, EVERIS®, NEUTRASE®, PROGRESS UNO®, RELASE® and ESPERASE® (Novozymes); BLAP™ and BLAP™ variants (Henkel); LAVERGY™ PRO 104 L (BASF), and KAP® (B. alkalophilus subtilisin) (Kao).Suitable proteases include naturally occurring proteases or engineered variants specifically selected or engineered to work at relatively low temperatures.

Lipases: Suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g.,* from *H. lanuginosa* (*T. lanuginosus*) (see *e.g.,* EP 258068 and EP 305216), from H. insolens (see *e.g.,* WO 96/13580); a *Pseudomonas* lipase (*e.g.,* from *P. alcaligenes* or *P. pseudoalcaligenes*; see, *e.g.,* EP 218 272), *P. cepacia* (see *e.g.,* EP 331 376), *P. stutzeri* (see *e.g.,* GB 1,372,034), *P. fluorescens*, *Pseudomonas* sp. strain SD 705 (see *e.g.,* WO 95/06720 and WO 96/27002), *P. wisconsinensis* (see *e.g.,* WO 96/12012); a *Bacillus* lipase (*e.g.,* from *B. subtilis*; see *e.g.,* Dartois et al. (1993) Biochemica et Biophysica Acta 1131:253-360), *B. stearothermophilus* (see *e.g.,* JP 64/744992), or *B. pumilus* (see *e.g.,* WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105.

Exemplary commercial lipases include, but are not limited to M1 LIPASE, LUMA FAST, and LIPOMAX (Genencor); LIPEX®, LIPOCLEAN®, LIPOLASE® and LIPOLASE® ULTRA (Novozymes); and LIPASE P (Amano Pharmaceutical Co. Ltd).

Polyesterases: Suitable polyesterases can be included in the composition, such as those described in, for example, WO 01/34899, WO 01/14629, and US6933140.

Amylases: The present compositions can be combined with other amylases, including other $\alpha$-amylases. Such a combination is particularly desirable when different $\alpha$-amylases demonstrate different performance characteristics and the combination of a plurality of different $\alpha$-amylases results in a composition that provides the benefits of the different $\alpha$-amylases. Other amylases include commercially available amylases, such as but not limited to STAINZYME®, NATALASE®, DURAMYL®, TERMAMYL®, FUNGAMYL® and BAN™ (Novo Nordisk A/S and Novozymes A/S); RAPIDASE®, POWERASE®, PURASTAR®, and PREFERENZ™ (from DuPont Industrial Biosciences.). Exemplary $\alpha$-amylases are described in WO9418314A1, US20080293607, WO2013063460, WO10115028, WO2009061380A2, WO2014099523, WO2015077126A1, WO2013184577, WO2014164777, WO9510603, WO9526397, WO9623874, WO9623873, WO9741213, WO9919467, WO0060060, WO0029560, WO9923211, WO9946399, WO0060058, WO0060059, WO9942567, WO0114532, WO02092797, WO0166712, WO0188107, WO0196537, WO0210355, WO2006002643, WO2004055178, and WO9813481.

Cellulases: Cellulases can be added to the compositions. Suitable cellulases include those of bacterial or fungal origin. Chemically modified or protein engineered mutants are included. Suitable cellulases include cellulases from the genera *Bacillus*, *Pseudomonas*, *Humicola*, Fusarium, *Thielavia*, *Acremonium*, *e.g.,* the fungal cellulases produced from *Humicola insolens*, *Myceliophthora thermophila* and *Fusarium* oxysporum disclosed for example in U.S. Patent Nos. 4,435,307; 5,648,263; 5,691,178; 5,776,757; and WO 89/09259. Exemplary cellulases contemplated for use are those having color care benefit for the textile. Examples of such cellulases are cellulases described in for example EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Exemplary cellulases include those described in WO2005054475, WO2005056787, US 7,449,318, US 7,833,773, US 4,435,307; EP 0495257; and US Provisional Appl. Nos. 62/296,678 and 62/435340. Exemplary commercial cellulases include, but are not limited to, CELLUCLEAN®, CELLUZYME®, CAREZYME®, CAREZYME® PREMIUM, ENDOLASE®, and RENOZYME® (Novozymes); REVITALENZ®100, REVITALENZ® 200/220 and REVITALENZ® 2000 (Danisco US); and KAC-500(B) (Kao Corporation).

Mannanases: Exemplary mannanases include, but are not limited to, those of bacterial or fungal origin, such as, for example, as is described in WO2016007929; USPNs 6566114, 6602842, and 6440991; and International Appl Nos. PCT/US2016/060850 and PCT/US2016/060844. Exemplary mannanases include, but are not limited to, those of bacterial or fungal origin, such as, for example, as is described in WO2016007929; USPNs 6566114, 6602842, and 6440991; and International Appl Nos. PCT/US2016/060850 and PCT/US2016/060844.

Peroxidases/Oxidases: Suitable peroxidases/oxidases contemplated for use in the compositions include those of plant, bacterial or fungal origin. Chemically modified or protein engineered mutants are included. Examples of useful peroxidases include peroxidases from Coprinus, *e.g.,* from *C. cinereus*, and variants thereof as those described in WO 93/24618, WO 95/10602, and WO 98/15257. Commercially available peroxidases include for example GUARDZYME™ (Novo Nordisk A/S and Novozymes A/S).

The detergent composition can also comprise 2,6-β-D-fructan hydrolase, which is effective for removal/cleaning of biofilm present on household and/or industrial textile/laundry.

The detergent enzyme(s) may be included in a detergent composition by adding separate additives containing one or more enzymes, or by adding a combined additive comprising all of these enzymes. A detergent additive, *i.e.* a separate additive or a combined additive, can be formulated, *e.g.*, as a granulate, a liquid, a slurry, and the like. Exemplary detergent additive formulations include but are not limited to granulates, in particular non-dusting granulates, liquids, in particular stabilized liquids or slurries.

Non-dusting granulates may be produced, *e.g.,* as disclosed in U.S. Patent Nos. 4,106,991 and 4,661,452 and may optionally be coated by methods known in the art. Examples of waxy coating materials are poly(ethylene oxide) products (*e.g.*, polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition may be in any convenient form, *e.g.*, a bar, a tablet, a powder, a granule, a paste, or a liquid. A liquid detergent may be aqueous, typically containing up to about 70% water, and 0% to about 30% organic solvent. Compact detergent gels containing about 30% or less water are also contemplated. The detergent composition can optionally comprise one or more surfactants, which may be non-ionic, including semi-polar and/or anionic and/or cationic and/or zwitterionic. The surfactants can be present in a wide range, from about 0.1% to about 60% by weight.

When included therein the detergent will typically contain from about 1% to about 40% of an anionic surfactant, such as linear alkylbenzenesulfonate, α-olefinsulfonate, alkyl sulfate (fatty alcohol sulfate), alcohol ethoxysulfate, secondary alkanesulfonate, α-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (*e.g.*, SKS-6 from Hoechst).

The detergent may comprise one or more polymers. Exemplary polymers include carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates *e.g.*, polyacrylates, maleic/acrylic acid copolymers), and lauryl methacrylate/acrylic acid copolymers.

The enzyme(s) of the detergent composition may be stabilized using conventional stabilizing agents, *e.g.,* as polyol (*e.g.*, propylene glycol or glycerol), a sugar or sugar alcohol, lactic acid, boric acid, or a boric acid derivative (*e.g.*, an aromatic borate ester), or a phenyl boronic acid derivative (*e.g.*, 4-formylphenyl boronic acid). The composition may be formulated as described in WO 92/19709 and WO 92/19708.

It is contemplated that in the detergent compositions, in particular the enzyme variants, may be added in an amount corresponding to about 0.01 to about 100 mg of enzyme protein per liter of wash liquor (*e.g.*, about 0.05 to about 5.0 mg of enzyme protein per liter of wash liquor or 0.1 to about 1.0 mg of enzyme protein per liter of wash liquor).

Numerous exemplary detergent formulations to which the present amylases can be added (or is in some cases are identified as a component of) are described in WO2013063460. These include commercially available unit dose detergent formulations/packages such as PUREX® UltraPacks (Henkel), FINISH® Quantum (Reckitt Benckiser), CLOROX™ 2 Packs (Clorox), OxiClean Max Force Power Paks (Church & Dwight), TIDE® Stain Release, CASCADE® ActionPacs, and TIDE® Pods (Procter & Gamble), PS.

### 7.6. Methods of Assessing Amylase Activity in Detergent Compositions

Numerous α-amylase cleaning assays are known in the art, including swatch and micro-swatch assays. The appended Examples describe only a few such assays.

In order to further illustrate the compositions and methods, and advantages thereof, the following specific examples are given with the understanding that they are illustrative rather than limiting.

### 8. Brewing Compositions

The present variant amylase may be a component of a brewing composition used in a process of brewing, *i.e.*, making a fermented malt beverage. Non-fermentable carbohydrates form the majority of the dissolved solids in the final beer. This residue remains because of the inability of malt amylases to hydrolyze the alpha-1,6-linkages of the starch. The non-fermentable carbohydrates contribute about 50 calories per 12 ounces of beer. An amylase, in combination with a glucoamylase and optionally a pullulanase and/or isoamylase, assists in converting the starch into dextrins and fermentable sugars, lowering the residual non-fermentable carbohydrates in the final beer.

### 9. Reduction of Iodine-Positive Starch

Variant amylases may reduce the iodine-positive starch (IPS), when used in a method of liquefaction and/or saccharification. One source of IPS is from amylose that escapes hydrolysis and/or from retrograded starch polymer. Starch retrogradation occurs spontaneously in a starch paste, or gel on ageing, because of the tendency of starch molecules to bind to one another followed by an increase in crystallinity. Solutions of low concentration become increasingly cloudy due to the progressive association of starch molecules into larger articles. Spontaneous precipitation takes place and the precipitated starch appears to be reverting to its original condition of cold-water insolubility. Pastes of higher concentration on cooling set to a gel, which on ageing becomes steadily firmer due to the increasing association of the starch molecules. This arises because of the strong tendency for hydrogen bond formation between hydroxy groups on adjacent starch molecules. See J.A. Radley, ed., Starch and its Derivatives 194-201 (Chapman and Hall, London (1968)). The use of variant amylases is expected to improve overall process performance by reducing the amount of IPS.

In order to further illustrate the compositions and methods, and advantages thereof, the following specific examples are given with the understanding that they are illustrative rather than limiting.

### EXAMPLE

### Generation of BspAmy24 variants

Proteins were prepared as follows. DNA sequences encoding the proteins of interest, a signal peptide for secretion, and additional 5' and 3' sequences for amplification and subcloning were ordered from a commercial vendor. Standard procedures were used to insert these DNA sequences into bacterial vectors for secreted protein expression in *Bacillus subtilis* cells. The constructs were verified by DNA sequencing. Cells were grown for 68 hours in expression medium suitable for secreted protein expression from *B. subtilis.* Cells were separated from protein-containing supernatant by centrifugation followed by filtration through 0.45 $\mu$m membranes (EMD Millipore). Additional purification was achieved through ion exchange chromatography with Phenyl Sepharose 6 Fast Flow resin (GE Healthcare). Protein concentration was determined by high performance liquid chromatography (HPLC) and absorbance at 280 nm. The variants of BspAmy24 (SEQ ID NO: 1) that were expressed and purified are shown in Table 2.

**Table 2.** BspAmy24 variants

| Variant | Mutations |
|---|---|
| BspAmy24-v1 | del (R181, G182) + T183D |
| BspAmy24-v2 | del (R181, G182) + T183D + E190P |
| BspAmy24-v3 | del (R181, G182) + T183D + M202L |
| BspAmy24-v4 | del (R181, G182) + T183D + E190P + M202L |
| BspAmy24-v5 | del (R181, G182) + Q172R + A186G + I324M |
| BspAmy24-v6 | del (T183, G184) + Q172R + A186G + I324M |
| BspAmy24-v7 | del (R181, G182) |

Starch cleaning activity of the variants was evaluated with rice starch on cotton commercial test fabric (CS28, Center for Test Materials, Netherlands) cut to 5.5 mm circular swatches. Two swatches were placed in each well of a 96-well Corning 9017 flat-bottomed polystyrene microtiter plate. Solutions of protein in commercial automatic dishwashing formulas with inactivated enzyme were added to the test fabric. The solution was shaken for 15 minutes at 50°C. Samples of the supernatant were evaluated for release of dye by measuring the absorbance of the solution at 488 nm with a spectrophotometer.

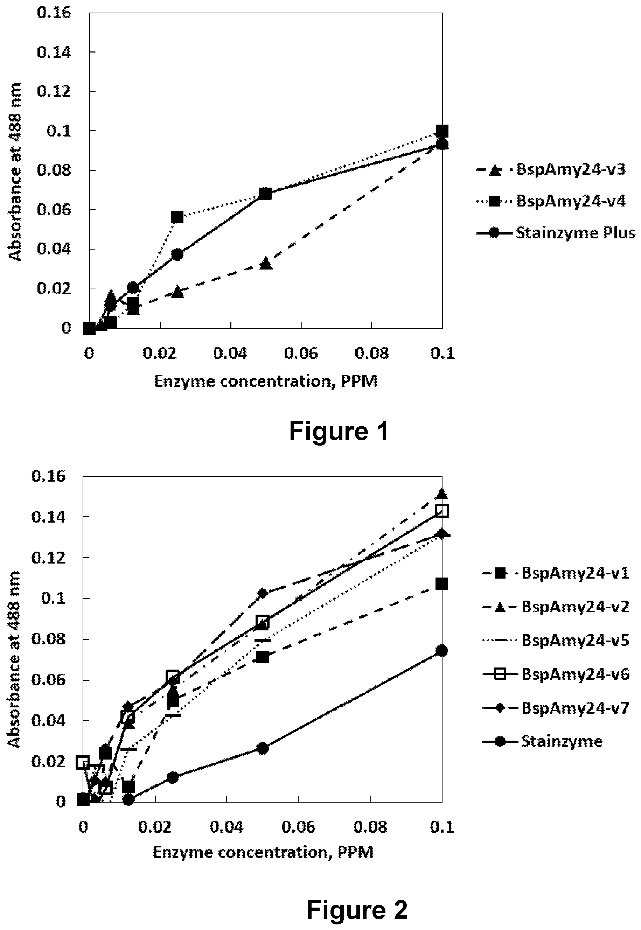

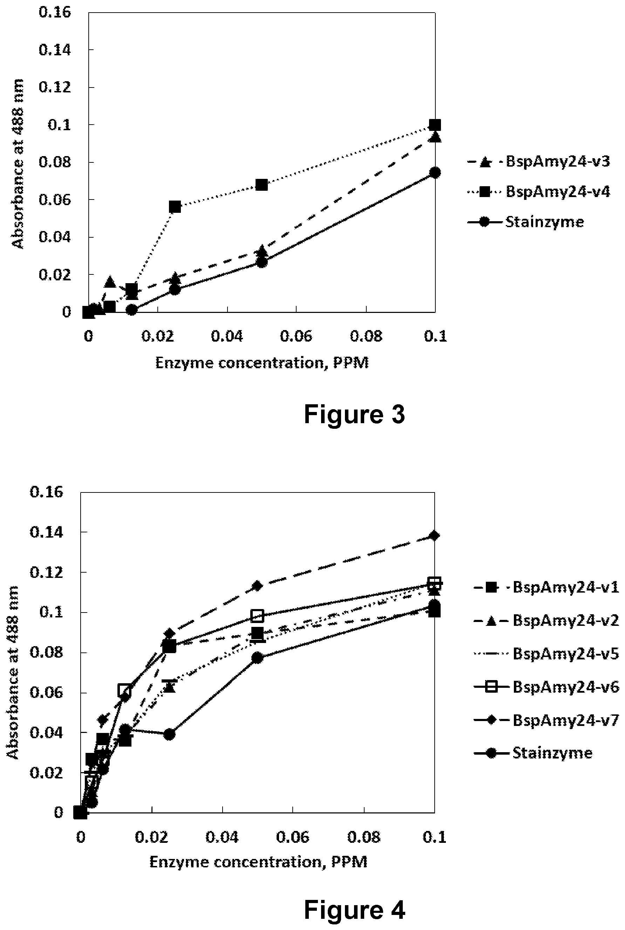

Commercial automatic dishwashing formulas with inactivated enzyme were prepared as follows. One individual dose packet of CASCADE® PLATINUM™ or FINISH® QUANTUM™ dishwashing formula was placed in 1 L of distilled water and stirred to dissolve the packet. The solution was incubated between 85 and 90°C for 10 hours. After cooling, the volume of the solution was raised to the approximate volume of a North American dishwasher (3.3 L) with distilled water and the water hardness was brought up to 150 PPM (8.76 GPG) with 3:1 calcium to magnesium ratio. The results of the cleaning assays are shown in Figures 1-4. The BspAmy24 variants performed well compared to STAINZYME® (Novozymes), a well-know cleaning amylase.

## Claims

1. A recombinant variant of a parent α-amylase comprising:

   (a) a deletion of amino acid residues corresponding to R181 and G182, using SEQ ID NO: 1; and the mutations Q172R, A186G and 1324M; wherein the variant α-amylase or the parent α-amylase has at least 91%, optionally 92%, optionally 93%, optionally 94%, optionally 95%, optionally 96%, optionally 97%, optionally 98%, or optionally 99%, amino acid sequence identity relative to SEQ ID NO: 1, which is used for numbering; and wherein the variant has improved cleaning performance in automatic dishwashing compared to the parent α-amylase,

   wherein said parent α-amylase differs from the variant α-amylase only by the absence of said deletion and mutations.

2. The variant α-amylase of claim 1, wherein the variant α-amylase has at least 95% amino acid sequence identity relative to SEQ ID NO: 1.

3. The variant α-amylase of claim 1, comprising the amino acid sequence of SEQ ID NO:1 with deletion of amino acid residues corresponding to R181 and G182 and the mutations Q172R, A186G and 1324M.

4. A polynucleotide encoding the variant amylase of any of claims 1-3, an expression vector comprising the polynucleotide, or an expression host comprising the polynucleotide or the expression vector.

5. A composition for liquefying starch comprising the variant amylase of any of claims 1-3.

6. A detergent composition comprising the variant amylase of any of claims 1-3.

7. A method for converting starch to oligosaccharides, comprising contacting starch with an effective amount of the variant amylase of any of the claims 1-3.

8. A method for removing a starchy stain or soil from a surface, comprising contacting the surface with an effective amount of the variant amylase of any of the claims 1-3, and allowing the polypeptide to hydrolyze starch components present in the starchy stain to produce smaller starch-derived molecules that dissolve in the aqueous composition, thereby removing the starchy stain from the surface.

## Patentansprüche

1. Rekombinante Variante einer Ausgangs-α-Amylase, umfassend:

   (a) eine Deletion von Aminosäureresten, die bei Verwendung der SEQ ID NO: 1 R181 und G182 entsprechen; und die Mutationen Q172R, A186G und I324M; wobei die α-Amylase-Variante oder die Ausgangs-α-Amylase mindestens 91 %, gegebenenfalls 92 %, gegebenenfalls 93 %, gegebenenfalls 94 %, gegebenenfalls 95 %, gegebenenfalls 96 %, gegebenenfalls 97 %, gegebenenfalls 98 % oder gegebenenfalls 99 % Aminosäuresequenzidentität im Vergleich zu SEQ ID NO: 1 aufweist, die zum Nummerieren verwendet wird; und wobei die Variante verbesserte Reinigungsleistung beim automatischen Geschirrspülen verglichen mit der Ausgangs-α-Amylase aufweist,

wobei sich die Ausgangs-α-Amylase von der α-Amylase-Variante nur durch das Fehlen der Deletion und von den Mutationen unterscheidet.

**2.** α-Amylase-Variante nach Anspruch 1, wobei die α-Amylase-Variante mindestens 95 % Aminosäuresequenzidentität im Vergleich zu SEQ ID NO: 1 aufweist.

**3.** α-Amylase-Variante nach Anspruch 1, umfassend die Aminosäuresequenz unter SEQ ID NO: 1 mit Deletion von Aminosäureresten, die R181 und G182 entsprechen, und mit den Mutationen Q172R, A186G und I324M.

**4.** Polynukleotid, codierend die Amylasevariante nach einem der Ansprüche 1-3, Expressionsvektor, umfassend das Polynukleotid, oder Expressionswirt, umfassend das Polynukleotid oder den Expressionsvektor.

**5.** Zusammensetzung zum Verflüssigen von Stärke, umfassend die Amylasevariante nach einem der Ansprüche 1-3.

**6.** Reinigungsmittelzusammensetzung, umfassend die Amylasevariante nach einem der Ansprüche 1-3.

**7.** Verfahren zum Umwandeln von Stärke in Oligosaccharide, umfassend Inkontaktbringen der Stärke mit einer wirksamen Menge der Amylasevariante nach einem der Ansprüche 1-3.

**8.** Verfahren zum Entfernen eines stärkehaltigen Flecks oder von Schmutz von einer Oberfläche, umfassend Inkontaktbringen der Oberfläche mit einer wirksamen Menge der Amylasevariante nach einem der Ansprüche 1-3 und Ermöglichen der Hydrolyse der Stärkekomponenten, die in dem stärkehaltigen Fleck vorhanden sind, durch das Polypeptid, sodass kleinere Stärke-abgeleitete Moleküle hergestellt werden, die sich in der wässrigen Zusammensetzung auflösen, wodurch der stärkehaltige Fleck von der Oberfläche entfernt wird.

**Revendications**

**1.** Variant recombinant d'une α-amylase parente comprenant :

(a) une délétion de résidus d'acides aminés correspondant à R181 et G182, en utilisant SEQ ID NO: 1 ; et les mutations Q172R, A186G et I324M ; dans lequel la α-amylase variante ou la α-amylase parente a au moins 91 %, éventuellement 92 %, éventuellement 93 %, éventuellement 94 %, éventuellement 95 %, éventuellement 96 %, éventuellement 97 %, éventuellement 98 % ou éventuellement 99 % d'identité de séquence d'acides aminés par rapport à SEQ ID NO: 1, qui est utilisée pour la numérotation ; et dans lequel la variante a des performances de nettoyage améliorées dans le lavage automatique de la vaisselle par rapport à la α-amylase parente,

dans lequel ladite α-amylase parente ne diffère de la α-amylase variante que par l'absence de ladite délétion et desdites mutations.

**2.** α-amylase variante selon la revendication 1, dans laquelle la α-amylase variante a une identité de séquence d'acides aminés d'au moins 95 % par rapport à SEQ ID NO: 1.

**3.** α-amylase variante selon la revendication 1, comprenant la séquence d'acides aminés de SEQ ID NO: 1 avec une délétion des résidus d'acides aminés correspondant à R181 et G182 et les mutations Q172R, A186G et I324M.

**4.** Polynucléotide codant pour l'amylase variante selon l'une quelconque des revendications 1 à 3, vecteur d'expression comprenant le polynucléotide, ou hôte d'expression comprenant le polynucléotide ou le vecteur d'expression.

**5.** Composition pour liquéfier de l'amidon comprenant l'amylase variante selon l'une quelconque des revendications 1 à 3.

**6.** Composition de détergent comprenant l'amylase variante selon l'une quelconque des revendications 1 à 3 .

**7.** Procédé pour convertir de l'amidon en oligosaccharides, comprenant la mise en contact de l'amidon avec une quantité efficace de l'amylase variante selon l'une quelconque des revendications 1 à 3.

**8.** Procédé pour éliminer une tache amidonnée ou une salissure d'une surface, comprenant la mise en contact de la surface avec une quantité efficace de l'amylase variante selon l'une quelconque des revendications 1 à 3, et le fait de laisser le polypeptide hydrolyser les composants amidon présents dans la tache amidonnée pour produire des molécules dérivées de l'amidon plus petites qui se dissolvent dans la composition aqueuse, éliminant ainsi la tache amidonnée de la surface.

Figure 1

Figure 2

**Figure 3**

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200164852 A1
- WO 2013063460 A2
- WO 2013184577 A1
- WO 2014164777 A1
- US 2016053243 A1
- US 5422267 A
- WO 2011153516 A2
- US 20060094080 A
- US 20070004018 A
- US 20070015266 A, Danisco US Inc.
- WO 9200381 A
- WO 0004136 A, Novo Nordisk A/S
- WO 8402921 A
- WO 9928448 A
- US 4587215 A
- EP 135138 A, CPC International, Inc.
- WO 8601831 A
- US 6077316 A
- US 4106991 A
- US 4661452 A
- GB 1483591 A
- EP 238216 A
- WO 9206154 A
- WO 2005056783 A
- WO 9219709 A
- WO 9219708 A
- US 20090209445 A1
- US 20100081598 A1
- US 7001878 B2
- EP 1504994 B1
- WO 2001085888 A2
- WO 2003089562 A1
- WO 2009098659 A1
- WO 2009098660 A1
- WO 2009112992 A1
- WO 2009124160 A1
- WO 2009152031 A1
- WO 2010059483 A1
- WO 2010088112 A1
- WO 2010090915 A1
- WO 2010135238 A1
- WO 2011094687 A1
- WO 2011094690 A1
- WO 2011127102 A1
- WO 2011163428 A1
- WO 2008000567 A1
- WO 2006045391 A1
- WO 2006007911 A1
- WO 2012027404 A1
- EP 1740690 B1
- WO 2012059336 A1
- US 6730646 B1
- WO 2008087426 A1
- WO 2010116139 A1
- WO 2012104613 A1
- WO 8906279 A
- WO 9523221 A
- WO 9221760 A
- WO 2008010925 A
- WO 20100566356 A
- WO 2011072099 A
- WO 201113022 A
- WO 2011140364 A
- WO 2012151534 A
- WO 2015038792 A
- WO 2015089441 A
- WO 2015089447 A
- WO 2015143360 A
- WO 2016001449 A
- WO 2016001450 A
- WO 2016061438 A
- WO 2016069544 A
- WO 2016069548 A
- WO 2016069552 A
- WO 2016069557 A
- WO 2016069563 A
- WO 2016069569 A
- WO 2016087617 A
- WO 2016087619 A
- WO 2016145428 A
- WO 2016174234 A
- WO 2016183509 A
- WO 2016202835 A
- WO 2016205755 A
- US 20080090747 A
- US 5801039 A
- US 5340735 A
- US 5500364 A
- US 5855625 A
- US 5955340 A
- US 5700676 A
- US 6312936 B
- US 6482628 B
- US 8530219 B
- US 62331282
- US 62343618 B
- US 62351649 B
- US 62437171 B

- US 62437174 B
- US 62437509 B
- CN 2017076749 W
- WO 2007044993 A
- WO 2009058303 A
- WO 2009058661 A
- WO 2014071410 A
- WO 2014194032 A
- WO 2014194034 A
- WO 2014194054 A
- WO 2014194117 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- WO 9535381 A
- WO 9600292 A
- WO 9530744 A
- WO 9425578 A
- WO 9514783 A
- WO 9522615 A
- WO 9704079 A
- WO 9707202 A
- EP 407225 A
- EP 260105 A
- WO 0134899 A
- WO 0114629 A
- US 6933140 B
- WO 9418314 A1
- US 20080293607 A
- WO 2013063460 A
- WO 10115028 A
- WO 2009061380 A2
- WO 2014099523 A
- WO 2015077126 A1
- WO 2013184577 A
- WO 2014164777 A
- WO 9510603 A
- WO 9526397 A
- WO 9623874 A
- WO 9623873 A
- WO 9741213 A

- WO 9919467 A
- WO 0060060 A
- WO 0029560 A
- WO 9923211 A
- WO 9946399 A
- WO 0060058 A
- WO 0060059 A
- WO 9942567 A
- WO 0114532 A
- WO 02092797 A
- WO 0166712 A
- WO 0188107 A
- WO 0196537 A
- WO 0210355 A
- WO 2006002643 A
- WO 2004055178 A
- WO 9813481 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- WO 9812307 A
- WO 9524471 A
- DK 9800299 W
- EP 531315 A
- US 5457046 A
- US 5686593 A
- US 5763254 A
- WO 2005054475 A
- WO 2005056787 A
- US 7449318 B
- US 7833773 B
- US 62296678
- US 62435340 B
- WO 2016007929 A
- US 6566114 B
- US 6602842 B
- US 6440991 B
- US 2016060850 W
- US 2016060844 W
- WO 9324618 A
- WO 9510602 A
- WO 9815257 A

**Non-patent literature cited in the description**

- **IGARAHI et al.** *Biochemical and Biophysical Research Communciations*, 1998, vol. 248, 372-377

- **THOMPSON et al.** *Nucleic Acids Res.*, 1994, vol. 22, 4673-4680

- **LIU et al.** *Sheng Wu Gong Cheng Xue Bao*, 2011, vol. 27, 1049-56
- **PAPAGIANNI.** *Biotechnol. Adv.*, 2007, vol. 25, 244-63
- **JOHN et al.** *Biotechnol. Adv.*, 2009, vol. 27, 145-52
- **BOEL et al.** *EMBO J.*, 1984, vol. 3, 1097-1102
- **CHEN et al.** *Prot. Eng.*, 1996, vol. 9, 499-505
- **CHEN et al.** *Prot. Eng.*, 1995, vol. 8, 575-582
- **CHEN et al.** *Biochem. J.*, 1994, vol. 301, 275-281
- **FIEROBE et al.** *Biochemistry*, 1996, vol. 35, 8698-8704
- **LI et al.** *Protein Eng.*, 1997, vol. 10, 1199-1204
- **FOGARTY et al.** *Progress in Industrial Microbiology*, 1979, vol. 15, 112-115
- **HAGE et al.** *Nature*, 1994, vol. 369, 637-639
- **DARTOIS et al.** *Biochemica et Biophysica Acta*, 1993, vol. 1131, 253-360
- **J.A. RADLEY.** Starch and its Derivatives. Chapman and Hall, 1968, 194-201